# EUROPÄISCHE PATENTSCHRIFT

(11) **EP 0 542 671 B1**
(45) Veröffentlichungstag und Bekanntmachung des Hinweises auf die Patenterteilung: **02.01.1997**
(21) Anmeldenummer: 92810768.9
(22) Anmeldetag: 08.10.1992
(51) Int. Cl.: C07C 43/23, C07D 211/62, C07F 9/54, C07F 9/40, C07D 405/04, C07C 69/78

(54) **Substituierte Hydrochinonderivate als Zwischenprodukte von Benzofuranderivaten**
Substituted hydroquinone derivatives as intermediates of benzofuran derivatives
Dérivés d'hydroquinone substitués comme intermédiaires pour la préparation de dérivés de benzofuranne

(30) Priorität: 17.10.1991 CH 3042/91
(43) Veröffentlichungstag der Anmeldung: 19.05.1993
(73) Patentinhaber: CIBA-GEIGY AG, 4002 Basel (CH)
(72) Erfinder: Sedelmeier, Gottfried, Dr., W-7801 Schallstadt (DE); Fischer, Gerhard, Dr., W-7812 Bad Krozingen (DE)

(56) Entgegenhaltungen:
- EP-A- 0 302 788
- DE-A- 2 653 147
- JOURNAL OF MEDICINAL CHEMISTRY. Bd. 23, Nr. 12, 1980, WASHINGTON US Seiten 1306 - 1310 C. GUEREMY ET AL. '3-(4-Piperidinylalkyl)indoles, Selective Inhibitors of Neuronal 5-Hydroxytryptamine Uptake'
- EUROPEAN JOURNAL OF MEDICINAL CHEMISTRY.CHIMICA THERAPEUTICA. Bd. 18, Nr. 5, 1983, CHATENAY-MALABRY FR Seiten 431 - 436 J. GUILLAUMEL ET AL. 'Recherches sur les dérivés nitrés d'intérêt biologique'

## Beschreibung

Die Erfindung betrifft neue substituierte Hydrochinonderivate der Formel I 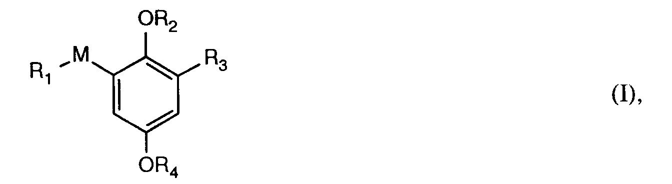 worin R₁ Hydroxy, Halogen, eine Gruppe der Formel -P(=O)(R₅)R₆ (Ia) oder eine Gruppe der Formel -P⁺(R₇)(R₈)R₉ X⁻ (Ib) ist, M Methylen ist, R₂ Wasserstoff oder eine Gruppe der Formel 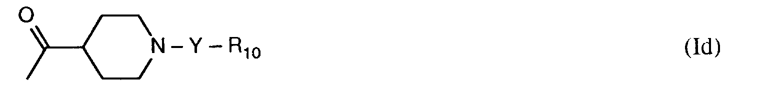 ist, R₃ Wasserstoff oder Halogen ist, R₄ Niederalkyl ist, R₅ und R₆ jeweils unabhängig voneinander Niederalkyl, Niederalkoxy, N,N-Diniederalkylamino oder jeweils unsubstituiertes oder an dem Phenylring ein- oder zweifach substituiertes Benzyl, Benzyloxy, Phenyl oder Phenyloxy darstellen, wobei die Substituenten des Phenylrings jeweils aus der Gruppe ausgewählt sein können, die aus Niederalkyl, Niederalkoxy, Nitro und Halogen besteht, R₇, R₈ und R₉ jeweils unabhängig voneinander Niederalkyl, unsubstituiertes oder ein- oder zweifach substituiertes Phenyl, wobei die Substituenten des Phenylrings jeweils aus der Gruppe ausgewählt sein können, die aus Niederalkyl, Niederalkoxy, Nitro und Halogen besteht, oder Furyl darstellen, X⁻ das Anion einer Halogenwasserstoffsäure, einer Niederalkansulfonsäure, einer Halogenniederalkansulfonsäure oder einer unsubstituierten oder durch Niederalkyl oder Halogen einfach substituierten Benzolsulfonsäure ist und worin entweder Y für eine direkte Bindung steht und R₁₀ Niederalkyl, Allyl, Cyano, Niederalkansulfonyl, Halogenniederalkansulfonyl, unsubstituiertes oder an dem Phenylring ein- oder zweifach substituiertes Benzyl, wobei die Substituenten des Phenylrings aus der Gruppe ausgewählt sein können, die aus Niederalkyl, Niederalkoxy, Nitro und Halogen besteht, oder unsubstituiertes oder durch Niederalkyl oder Halogen einfach substituiertes Benzolsulfonyl ist oder worin Y eine Gruppe der Formel -(C=O)- (Ie) oder eine Gruppe der Formel -(C=S)- (If) ist und R₁₀ Halogen, Niederalkyl, Halogenniederalkyl, Niederalkoxy, Niederalkenyloxy, Phenylniederalkenyloxy, Halogenniederalkoxy, Niederalkylthio oder jeweils unsubstituiertes oder an dem Phenylring ein- oder zweifach substituiertes Phenyl, Phenyloxy, Phenylthio, Benzyl, Benzyloxy oder Benzylthio ist, wobei die Substituenten des Phenylrings jeweils aus der Gruppe ausgewählt sein können, die aus Niederalkyl, Niederalkoxy, Nitro und Halogen besteht, mit der Massgabe, dass in einer Verbindung I, worin R₁ eine Gruppe Ib, worin R₇, R₈ und R₉ jeweils unsubstituiertes Phenyl bedeuten und X⁻ das Bromidion ist, R₂ Wasserstoff ist und R₄ Methyl ist, R₃ von Wasserstoff verschieden ist, und mit der weiteren Massgabe, dass in einer Verbindung I, worin R₁ Hydroxy ist, R₂ Wasserstoff ist und R₄ Methyl oder Ethyl ist, R₃ von Wasserstoff verschieden ist, in freier Form oder in Salzform, ein Verfahren zur Herstellung und die Verwendung dieser Verbindungen und ein Verfahren, in dem diese Verbindungen verwendet werden.

Verbindungen I in Salzform sind insbesondere entsprechende Säureadditionssalze (von Verbindungen I mit mindestens einem basischen Zentrum) und entsprechende Salze mit Basen (von Verbindungen I mit mindestens einem sauren Zentrum), vorzugsweise entsprechende pharmazeutisch verwendbare Salze. Die Säureadditionssalze werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie Niederalkancarbonsäuren, z.B. Essigsäure, wie gegebenenfalls ungesättigten Dicarbonsäuren, z.B. Oxal-, Malon-, Bernstein-, Malein-, Fumar-, Phthal- oder Terephthalsäure, wie Hydroxycarbonsäuren, z.B. Ascorbin-, Glykol-, Milch-, Äpfel-, Wein- oder Zitronensäure, wie Aminosäuren, z.B. Asparagin- oder Glutaminsäure, oder wie Benzoesäure, oder mit organischen Sulfonsäuren, wie Niederalkan- oder gegebenenfalls substituierten Benzolsulfonsäuren, z.B. Methan- oder p-Toluolsulfonsäure, gebildet. Geeignete Salze mit Basen sind beispielsweise Metallsalze, wie Alkali- oder Erdalkalimetallsalze, z.B. Natrium-, Kalium- oder Magnesiumsalze, oder Salze mit Ammoniak oder einem organischen Amin, wie Morpholin, Thiomorpholin, Piperidin, Pyrrolidin, einem Mono-, Di- oder Triniederalkylamin, z.B. Ethyl-, tert.-Butyl-, Diethyl-, Diisopropyl-, Triethyl-, Tributyl- oder Dimethylpropylamin, oder einem Mono-, Di- oder Trihydroxyniederalkylamin, z.B. Mono-, Di- oder Triethanolamin. Weiterhin können die Verbindungen I gegebenenfalls als innere Salze vorliegen, z.B. dann, wenn R₁ eine Gruppe Ib ist. Umfasst sind ferner für pharmazeutische Verwendungen ungeeignete Salze, die beispielsweise für die Isolierung bzw. Reinigung freier Verbindungen I sowie deren pharmazeutisch verwendbarer Salze verwendet werden können. Infolge der engen Beziehung zwischen den Verbindungen I in freier Form und in Form ihrer Salze sind vorstehend und nachfolgend unter den freien Verbindungen I bzw. ihren Salzen sinn- und zweckgemäss gegebenenfalls auch die entsprechenden Salze bzw. die freien Verbindungen I zu verstehen.

Die vor- und nachstehend verwendeten Allgemeinbegriffe haben, sofern nicht abweichend definiert, die nachfolgend aufgeführten Bedeutungen.

Unter mit "Nieder" bezeichneten Resten und Verbindungen sind, sofern nicht abweichend definiert, solche zu verstehen, die bis und mit 7, vor allem bis und mit 4, Kohlenstoffatome enthalten.

Niederalkyl ist C₁-C₄-Alkyl, d.h. Methyl, Ethyl, Propyl, Isopropyl, Butyl, Isobutyl, sek.-Butyl oder tert.-Butyl, und umfasst ferner C₅-C₇-Alkyl-, d.h. entsprechende Pentyl-, Hexyl- und Heptylreste.

Niederalkoxy ist C₁-C₄-Alkoxy, d.h. Methoxy, Ethoxy, Propyloxy, Isopropyloxy, Butyloxy, Isobutyloxy, sek.-Butyloxy oder tert.-Butyloxy, und umfasst ferner C₅-C₇-Alkoxy-, d.h. entsprechende Pentyloxy-, Hexyloxy- und Heptyloxyreste.

Niederalkenyloxy ist beispielsweise Allyloxy; Phenylniederalkenyloxy bedeutet beispielsweise 3-Phenylprop-2-enyl.

Niederalkylthio ist C₁-C₄-Alkylthio, d.h. Methylthio, Ethylthio, Propylthio, Isopropylthio, Butylthio, Isobutylthio, sek.-Butylthio oder tert.-Butylthio, und umfasst ferner C₅-C₇-Alkylthio-, d.h. entsprechende Pentylthio-, Hexylthio- und Heptylthioreste.

Halogen ist insbesondere Chlor oder Brom, ferner Fluor oder Iod.

N,N-Diniederalkylamino ist solches, worin die beiden N-Niederalkylreste gleich oder verschieden sind und jeweils die vorstehend bei der Definition von Niederalkyl angegebene Bedeutung haben, wie N,N-Dimethylamino, N,N-Diethylamino, N,N-Diisopropylamino, N-Butyl-N-methyl-amino, N,N-Dipentylamino oder N-Pentyl-N-methyl-amino.

Halogenniederalkyl enthält - verglichen mit Niederalkyl - an einem seiner C-Atome ein, zwei oder drei gleiche oder verschiedene Halogenatome anstelle von Wasserstoffatomen und ist solches, worin Niederalkyl die vorstehend bei der Definition von Niederalkyl angegebene Bedeutung und Halogen die vorstehend bei der Definition von Halogen angegebene Bedeutung hat, wie Trifluormethyl, Trichlormethyl, 2,2,2-Trifluorethyl oder 3,3,3-Trifluorpropyl.

Halogenniederalkoxy enthält - verglichen mit Niederalkoxy - an einem seiner C-Atome ein, zwei oder drei gleiche oder verschiedene Halogenatome anstelle von Wasserstoffatomen und ist solches, worin Niederalkoxy die vorstehend bei der Definition von Niederalkoxy angegebene Bedeutung und Halogen die vorstehend bei der Definition von Halogen angegebene Bedeutung hat, wie Trifluormethoxy, Trichlormethoxy, 2-Iodethoxy, 2,2,2-Trifluorethoxy oder 3,3,3-Trifluorpropyloxy.

Niederalkansulfonyl ist C₁-C₄-Alkansulfonyl, wie Methansulfonyl, Ethansulfonyl, Propansulfonyl, Butansulfonyl oder tert.-Butansulfonyl, und umfasst ferner C₅-C₇-Alkansulfonyl-, d.h. entsprechende Pentansulfonyl-, Hexansulfonyl- und Heptansulfonylreste.

Halogenniederalkansulfonyl enthält - verglichen mit Niederalkansulfonyl - an einem seiner C-Atome ein, zwei oder drei gleiche oder verschiedene Halogenatome anstelle von Wasserstoffatomen und ist solches, worin Niederalkansulfonyl die vorstehend bei der Definition von Niederalkansulfonyl angegebene Bedeutung und Halogen die vorstehend bei der Definition von Halogen angegebene Bedeutung hat, wie Trifluormethansulfonyl, Trichlormethansulfonyl, 2,2,2-Trifluorethansulfonyl oder 3,3,3-Trifluorpropansulfonyl.

Anionen einer Halogenwasserstoffsäure (Halogenidionen) sind insbesondere das Chlorid- und das Bromidion, ferner das Fluorid- und das Iodidion.

Anionen einer Niederalkansulfonsäure (Niederalkansulfonationen) sind solche, worin das ihrer Niederalkansulfonat-Struktur zugrundeliegende Niederalkansulfonyl die vorstehend bei der Definition von Niederalkansulfonyl angegebene Bedeutung hat, wie das Methansulfonation, das Ethansulfonation, das Propansulfonation, das Butansulfonation oder das tert.-Butansulfonation.

Anionen einer Halogenniederalkansulfonsäure (Halogenniederalkansulfonationen) enthalten - verglichen mit Anionen einer Niederalkansulfonsäure (Niederalkansulfonationen) - an einem ihrer C-Atome ein, zwei oder drei gleiche oder verschiedene Halogenatome anstelle von Wasserstoffatomen und sind solche, worin das ihrer Niederalkansulfonat-Teilstruktur zugrundeliegende Niederalkansulfonyl die vorstehend bei der Definition von Niederalkansulfonyl angegebene Bedeutung und Halogen die vorstehend bei der Definition von Halogen angegebene Bedeutung hat, wie das Trifluormethansulfonation, das Trichlormethansulfonation, das 2,2,2-Trifluorethansulfonation oder das 3,3,3-Trifluorpropansulfonation.

Anionen einer unsubstituierten oder durch Niederalkyl oder Halogen einfach substituierten Benzolsulfonsäure (unsubstituierte oder durch Niederalkyl oder Halogen einfach substituierte Benzolsulfonationen) sind solche, worin Niederalkyl die vorstehend bei der Definition von Niederalkyl angegebene Bedeutung und Halogen die vorstehend bei der Definition von Halogen angegebene Bedeutung hat, wie das Benzolsulfonation, das p-Toluolsulfonation oder das p-Chlorbenzolsulfonation.

Bevorzugt sind im Rahmen der Erfindung Verbindungen der Formel I, worin R₁ Hydroxy, Halogen, eine Gruppe Ia oder eine Gruppe Ib ist, M Methylen ist, R₂ Wasserstoff oder eine Gruppe Id ist, R₃ Wasserstoff oder Halogen ist und R₄ Niederalkyl ist, R₅ und R₆ jeweils unabhängig voneinander Niederalkoxy darstellen, R₇, R₈ und R₉ jeweils unsubstituiertes Phenyl oder Furyl darstellen, X⁻ das Anion einer Halogenwasserstoffsäure ist, Y eine Gruppe Ie ist und R₁₀ Niederalkoxy oder Niederalkenyloxy ist, mit der Massgabe, dass in einer Verbindung I, worin R₁ eine Gruppe Ib bedeutet, in der X⁻ das Bromidion ist, ist, M Methylen ist, R₂ Wasserstoff ist und R₄ Methyl ist, R₃ von Wasserstoff verschieden ist, und mit der weiteren Massgabe, dass in einer Verbindung I, worin R₁ Hydroxy ist, M Methylen ist, R₂ Wasserstoff ist und R₄ Methyl oder Ethyl ist, R₃ von Wasserstoff verschieden ist, in freier Form oder in Salzform.

Ganz besonders bevorzugt sind im Rahmen der Erfindung Verbindungen der Formel I, worin R₁ Hydroxy oder eine Gruppe Ib ist, M Methylen ist, R₂ Wasserstoff oder eine Gruppe Id ist, R₃ Halogen ist, R₄ C₁-C₄-Alkyl ist, R₇, R₈ und R₉ jeweils unsubstituiertes Phenyl darstellen, X⁻ das Anion einer Halogenwasserstoffsäure ist, Y eine Gruppe Ie ist und R₁₀ C₁-C₄-Alkoxy ist, in freier Form oder in Salzform.

In ganz besonderer Weise bevorzugt sind im Rahmen der Erfindung Verbindungen der Formel I, worin R₁ eine Gruppe Ib ist, M Methylen ist, R₂ Wasserstoff oder eine Gruppe Id ist, R₃ Brom ist, R₄ Methyl ist, R₇, R₈ und R₉ jeweils unsubstituiertes Phenyl darstellen, X⁻ das Chloridion oder das Bromidion ist, Y eine Gruppe Ie ist und R₁₀ Ethoxy ist, in freier Form oder in Salzform.

Namentlich bevorzugt sind im Rahmen der Erfindung die in den Beispielen genannten Verbindungen der Formel I, in freier Form oder in Salzform.

Weiterer Gegenstand der Erfindung ist das Verfahren zur Herstellung der Verbindungen der Formel I, in freier Form oder in Salzform, dadurch gekennzeichnet, dass man
a) zur Herstellung einer Verbindung der Formel I, worin R₁ Niederalkoxy ist, M zusätzlich Carbonyl bedeutet, R₂ Wasserstoff, R₃ Halogen und R₄ Niederalkyl ist, oder eines Salzes davon eine Verbindung der Formel II 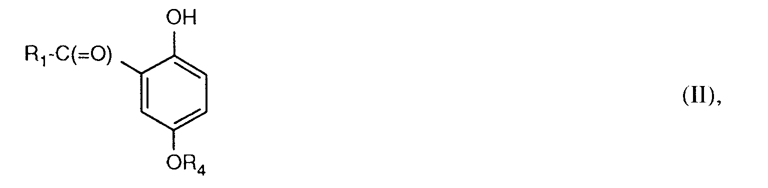 worin R₁ Hydroxy oder Niederalkoxy und R₄ Niederalkyl bedeutet, oder ein Salz davon durch Behandlung mit elementarem Halogen in einem Niederalkanol in m-Stellung zur R₁-C(=O)-Gruppe halogeniert, wobei Carboxy R₁-C(=O)-, sofern vorhanden, zu Niederalkoxycarbonyl verestert wird und/oder
b) zur Herstellung einer Verbindung der Formel I, worin R₁ Hydroxy, M Methylen, R₂ Wasserstoff, R₃ Wasserstoff oder Halogen und R₄ Niederalkyl ist, mit der Massgabe, dass R₃ von Wasserstoff verschieden ist, wenn R₄ Methyl oder Ethyl darstellt, oder eines Salzes davon eine Verbindung der Formel 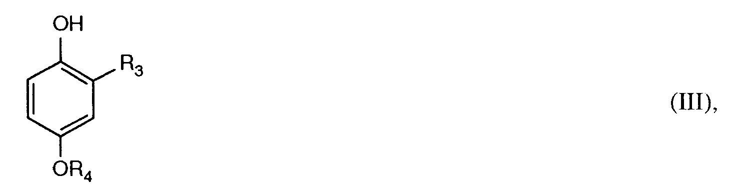 worin R₃ Wasserstoff oder Halogen und R₄ Niederalkyl ist, oder ein Salz davon mit Paraformaldehyd oder Trioxan umsetzt oder in einer Verbindung der Formel 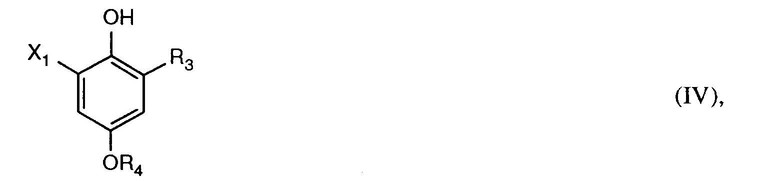 worin X₁ die Formylgruppe, Carboxy oder Niederalkoxycarbonyl bedeutet, R₃ Halogen und R₄ Niederalkyl ist, oder einem Salz davon die Gruppe X₁ zu Hydroxymethyl reduziert und/oder
c) zur Herstellung einer Verbindung der Formel I, worin R₁ Halogen, M Methylen, R₂ Wasserstoff und R₃ Halogen ist, oder eines Salzes davon in einer Verbindung der Formel 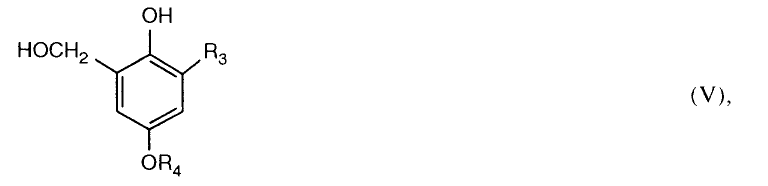 die Hydroxymethylgruppe in Halogenmethyl überführt oder eine Verbindung der Formel 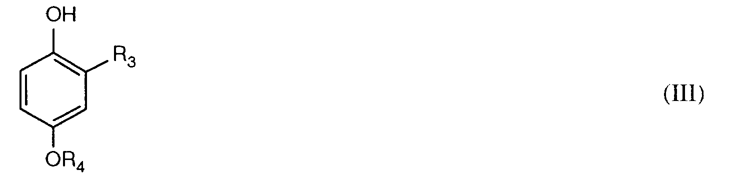 mit Paraformaldehyd oder Trioxan und einer Halogenwasserstoffsäure umsetzt und/oder
d) zur Herstellung einer Verbindung der Formel I, worin R₁ eine eine Gruppe der Formel -P(=O)(R₅)R₆ (Ia) oder eine Gruppe der Formel -P⁺(R₇)(R₈)R₉ X⁻ (Ib), M Methylen, R₂ Wasserstoff, R₃ Halogen und R₄ Niederalkyl ist und R₅, R₆, R₇, R₈, R₉ und X⁻ die unter der Formel I angegebenen Bedeutungen haben, oder eines Salzes davon eine Verbindung der Formel 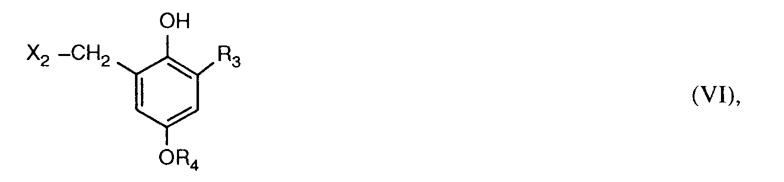 worin X₂ Halogen, R₃ Halogen und R₄ Niederalkyl ist, mit einer Verbindung der Formeln VIIa, VIIb oder VIIc 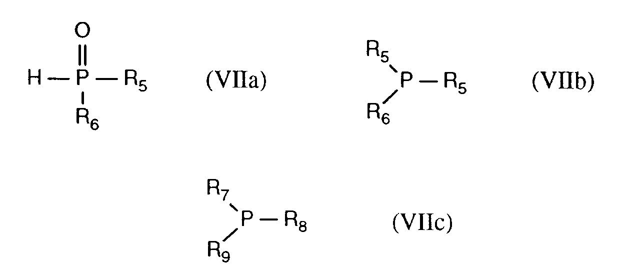 oder einem Salz davon umsetzt, worin R₅, R₆, R₇, R₈ und R₉ die unter der Formel I angegebenen Bedeutungen haben oder eine Verbindung der Formel 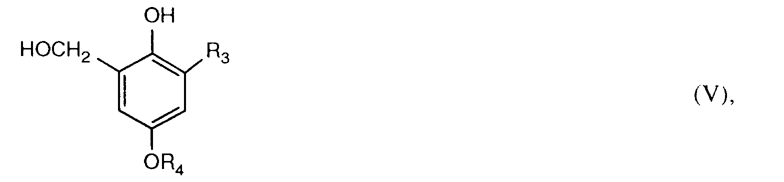 worin R₃ Halogen und R₄ Niederalkyl ist, mit einer Verbindung der Formel VIIc 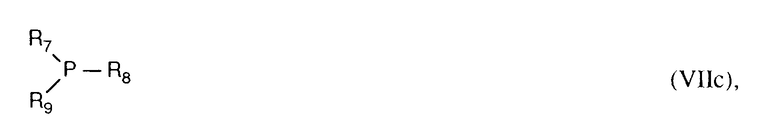 worin R₇, R₈ und R₉ die unter der Formel I angegebenen Bedeutungen haben, und einer Halogenwasserstoffsäure der Formel HX umsetzt und/oder
e) zur Herstellung einer Verbindung der Formel I, worin R₁ eine Gruppe der Formel -P(=O)(R₅)R₆ (Ia) oder eine Gruppe der Formel-P⁺(R₇)(R₈)R₉ X⁻(Ib), M Methylen, R₂ Wasserstoff, R₃ Halogen und R₄ Niederalkyl ist und R₅, R₆, R₇, R₈, R₉ und X⁻ die unter der Formel I angegebenen Bedeutungen haben, oder eines Salzes davon eine Verbindung der Formel 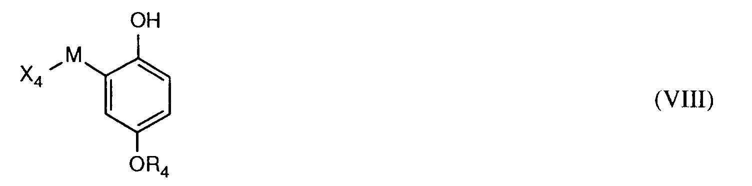 worin X₄ eine Gruppe der Formel -P(=O)(R₅)R₆ (Ia) oder eine Gruppe der Formel -P⁺(R₇)(R₈)R₉ X⁻ (Ib), M Methylen und R₄ Niederalkyl ist, in m-Stellung zur X₄-M-Gruppe halogeniert und/oder
f) zur Herstellung einer Verbindung der Formel I, worin R₁ eine Gruppe der Formel -P⁺(R₇)(R₈)R₉ X⁻ (Ib), M Methylen, R₂ eine Gruppe der Formel 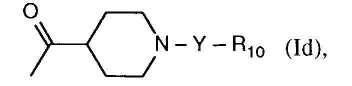 R₃ Wasserstoff oder Halogen und R₄ Niederalkyl ist und R₅, R₆, R₇, R₈, R₉, R₁₀, Y und X⁻die unter der Formel I angegebenen Bedeutungen haben, oder eines Salzes davon eine Verbindung der Formel 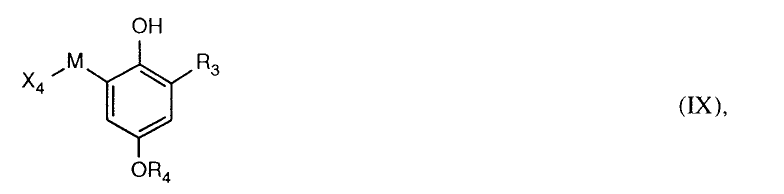 worin X₄ eine Gruppe der Formel -P⁺(R₇)(R₈)R₉ X⁻ (Ib), M Methylen, R₃ Halogen und R₄ Niederalkyl ist, und R₇, R₈, R₉ und X⁻ die unter der Formel I angegebenen Bedeutungen haben, mit einer Verbindung der Formel 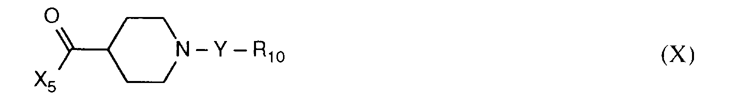 oder einem Salz davon umsetzt, worin X₅ Halogen oder eine Gruppe der Formel X₇-O- darstellt, in der X₇ Niederalkansulfonyl, Niederalkoxycarbonyl oder Niederalkanoyl ist oder eine Gruppe der Formel 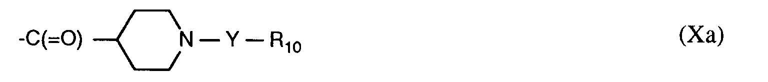 bedeutet, und R₁₀ und Y die unter der Formel I angegebenen Bedeutungen haben und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

Die Durchführung der verfahrensgemässen Reaktionen sowie die Herstellung neuer Ausgangsstoffe bzw. Zwischenprodukte erfolgt in Analogie zur Reaktions- und Bildungsweise bekannter Ausgangsstoffe bzw. Zwischenprodukte. Dabei werden, auch wenn nachstehend nicht ausdrücklich erwähnt, die jeweils üblichen Hilfsmittel, wie Katalysatoren, Kondensations- sowie Solvolysemittel und/oder Lösungs- bzw. Verdünnungsmittel, und Reaktions-, wie Temperatur- und Druckbedingungen, sowie gegebenenfalls Schutzgase verwendet.

Die Halogenierung von Verbindungen der Formel II gemäss Verfahrensschritt a) erfolgt beispielsweise durch Umsetzung mit elementaren, Halogen, insbesondere Brom, vorteilhaft in einem organischen Lösungsmittel, beispielsweise in einem aliphatischen Alkohol, insbesondere einem Niederalkanol, wie Methanol, Ethanol,Propanol, Isopropanol. Die Halogenierung wird vorzugsweise bei normaler oder geringfügig erniedrigter Temperatur, beispielsweise im Temperaturbereich von etwa 0 bis etwa 30° C, insbesondere von etwa 0° bis etwa 20° C , durchgeführt. Ausgehend von Carbonsäuren II wird dabei auch die Carboxygruppe in erwünschter Weise zu der entsprechenden Niederalkoxycarbonylgruppe verestert.

Die Ausgangsstoffe für den Verfahrensschritt a) sind bekannt.

Die Umsetzung von Verbindungen der Formel III mit Paraformaldehyd oder Trioxan gemäss Verfahrensschritt b) erfolgt beispielsweise in Gegenwart eines sauren Kondensationsmittels, wie einer schwachen Lewissäure, insbesondere von wasserfreier Borsäure. Diese wird vorzugsweise durch azeotrope Destillation mit einem Lösungsmittel, welches mit Wasser ein azeotropes Gemisch bildet, beispielsweise mit Toluol, *in situ* erzeugt. Vorzugsweise arbeitet man bei erhöhter Temperatur, beispielsweise im Temperaturbereich von etwa 60° bis etwa 120° C, insbesondere von etwa 80° bis etwa 100° C.

Die Reduktion von Formyl, Carboxy bzw. Niederalkoxycarbonyl X₁ in Verbindungen der Formel IV wird vorzugsweise durch Umsetzung mit einem Dileichtmetallhydrid, beispielsweise mit Lithiumaluminiumhydrid oder insbesondere Natriumborhydrid bewirkt. Vorzugsweise arbeitet man in einem etherischen Lösungsmittel, wie einen, aliphatischen oder cycloaliphatischen Ether, z.B. in Diethylether, Methoxybutan, Tetrahydrofuran oder Dioxan, oder bei der Verwendung von Natrumborhydrid in einem aliphatischen Alkohol, wie einem Niederalkanol, beispielsweise in Methanol oder Ethanol, bei normaler oder vorteilhaft leicht erniedrigter Temperatur, beispielsweise im Temperaturbereich von etwa 0° bis etwa 40° C, insbesondere von etwa 5° bis etwa 20° C.

Ausgangsstoffe der Formel III sowie Verbindungen der Formel IV, worin X₁ Formyl ist, sind bekannt; Ausgangsstoffe der Formel IV, worin X₁ Carboxy oder Niederalkoxycarbonyl ist, werden vorzugsweise gemäss Verfahrensschritt a) hergestellt.

Die Überführung von Hydroxymethyl in Halogenmethyl gemäss Verfahrensschritt c) erfolgt in üblicher Weise, beispielsweise durch Umsetzung mit einer Halogenwasserstoffsäure, wie Chlor- oder insbesondere Bromwasserstoff, oder einer Halogenwasserstoff-liefernden Verbindung. Als solche kommen beispielsweise Ammoniumhalogenide, wie Ammoniumbromid, oder im Hinblick auf die Weiterverwendung im Verfahrensschritt d) insbesondere Phophoniumhalogenide, wie Verbindungen der Formel HP+(R₇)(R₈)R₉X-, in Betracht. Vorteilhaft arbeitet man in einem organischen Lösungsmittel, wie einer aliphatischen Carbonsäure oder einen, davon abgeleiteten Niederalkylester oder Nitril, beispielsweise in Essigsaure, Essigsäureethylester oder Acetonitril. Die Wahl des Lösungsmittels ist indes nicht kritisch; man kann z.B. auch Toluol oder Benzol verwenden.

Ausgangsstoffe der Formel V, worin R₃ Wasserstoff und R₄ Methyl oder Ethyl ist, sind bekannt; weitere Verbindungen der Formel V können z.B. gemäss Verfahrensschritt b) hergestellt werden.

Die Umsetzung von Zwischenprodukten der Formel VI mit Verbindungen der Formeln VIIa, VIIb bzw. VIIc gemäss Verfahrensschritt d) erfolgt in üblicher Weise. So wird die Umsetzung von Verbindungen der Formel VI mit Verbindungen der Formeln VIIa bzw. VIIb vorzugsweise bei erhöhter Temperatur, beispielsweise im Temperaturbereich von etwa 100° bis etwa 200° C, vorzugsweise von etwa 120° bis etwa 140° C, durchgeführt (Verfahrensweise von Michaelis-Arbusow).

Die Umsetzung von Verbindungen der Formel VI mit Verbindungen der Formel VIIc erfolgt vorzugsweise in Gegenwart eines organischen Lösungs- oder Verdünnungsmittels, wie eines Niederalkancarbonsäureesters oder Niederalkancarbonsäurenitrils, wie Essigsäureethylester oder Acetonitril, erforderlichenfalls unter Erwärmen, z.B. im Temperaturbereich von etwa 30° bis etwa 100° C, vorzugsweise von etwa 40° bis etwa 80° C.

In einer bevorzugten, die Verfahrensschritte c) und d) zusammenfassenden Ausführungsform wird das Zwischenprodukt der Formel VI dabei vorteilhaft aus der entsprechenden Verbindung der Formel V durch Einwirkung einer Halogenwasserstoffsäure, z.B. von Chlorwasserstoff, oder indem man die Verbindung der Formel VIIc in Form eines Halogenwasserstoffsalzes einsetzt, *in situ* hergestellt und ohne Isolierung weiterumgesetzt.

Die Halogenierung von Verbindungen der Formel VIII gemäss Verfahrensschritt e) erfolgt beispielsweise durch Umsetzung mit elementaren Halogen, insbesondere Brom, vorteilhaft in einem organischen Lösungsmittel, beispielsweise in einem aliphatischen Alkohol, insbesondere einem Niederalkanol, wie Methanol, Ethanol,Propanol, Isopropanol.

Ausgangsstoffe der Formel VIII können beispielsweise gemäss Verfahrensschritt d) oder c)+d) hergestellt werden.

Die Umsetzung von Verbindungen der Formeln IX und X gemäss Verfahrensschritt f) erfolgt vorzugsweise in Gegenwart eines basischen Kondensationsmittels,wie einer organischen Stickstoffbase, wie einer aromatischen Stickstoffbase, z.B. von Pyridin oder Chinolin, oder eines aliphatischen Amins, insbesondere eines Triniederalkylamins, wie Triethylamin, vorteilhaft in einem organischen Lösungsmittel, beispielsweise in einem Niederalkancarbonsäureester oder Niederalkancarbonsäurenitril, wie Essigsäureethylester oder Acetonitril, erforderlichenfalls unter Erwärmen, z.B. im Temperaturbereich von etwa 25° bis etwa 100° C, vorzugsweise von etwa 40° bis etwa 80° C. X₅ bedeutet als Halogen insbesondere Chlor oder Brom, als Niederalkansulfonyloxy insbesondere Methansulfonyloxy und als Niederalkoxycarbonyloxy insbesondere Ethoxy- oder Tertiärbutoxycarbonyloxy.

Verfahrensgemäss erhältliche Verbindungen können in üblicher Weise in andere Verbindungen der Formel I überführt werden.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder 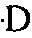 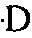 - oder 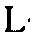 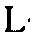 -(1-Phenyl)ethylamin, 3-Pipecolin, Ephedrin, Amphetamin und ähnliche synthetisch zugängliche Basen, optisch aktive Carbon- oder Sulfonsäuren, wie Chinasäure oder 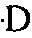 - oder 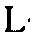 -Weinsäure, 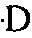 - oder 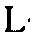 -Di-o-toluylweinsäure, 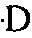 - oder 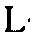 -Äpfelsäure, 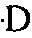 - oder 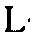 -Mandelsäure, oder 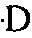 - oder 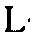 -Camphersulfonsäure, bzw. optisch aktive Alkohole, wie Borneol oder 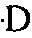 - oder 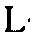 -(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die Verbindungen der Formel I, worin R₁ Hydroxy, Halogen, eine Gruppe der Formel -P(=O)(R₅)R₆, (Ia) oder eine Gruppe der Formel -P⁺(R₇)(R₈)R₉ X⁻ (Ib) ist, M Methylen ist, R₂ Wasserstoff oder eine Gruppe der Formel 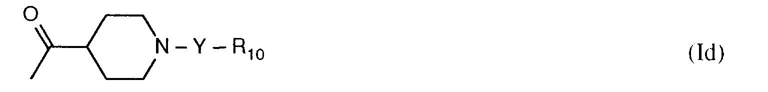 ist, R₃ Wasserstoff oder Halogen ist, R₄ Niederalkyl ist R₅ und R₆ jeweils unabhängig voneinander Niederalkyl, Niederalkoxy, N,N-Diniederalkylamino oder jeweils unsubstituiertes oder an dem Phenylring ein- oder zweifach substituiertes Benzyl, Benzyloxy, Phenyl oder Phenyloxy darstellen, wobei die Substituenten des Phenylrings jeweils aus der Gruppe ausgewählt sein können, die aus Niederalkyl, Niederalkoxy, Nitro und Halogen besteht, R₇, R₈ und R₉ jeweils unabhängig voneinander Niederalkyl, unsubstituiertes oder ein- oder zweifach substituiertes Phenyl, wobei die Substituenten des Phenylrings jeweils aus der Gruppe ausgewählt sein können, die aus Niederalkyl, Niederalkoxy, Nitro und Halogen besteht, oder Furyl darstellen, X⁻ das Anion einer Halogenwasserstoffsäure, einer Niederalkansulfonsäure, einer Halogenniederalkansulfonsäure oder einer unsubstituierten oder durch Niederalkyl oder Halogen einfach substituierten Benzolsulfonsäure ist und worin entweder Y für eine direkte Bindung steht und R₁₀ Niederalkyl, Allyl, Cyano, Niederalkansulfonyl, Halogoenniederalkansulfonyl, unsubstituiertes oder an dem Phenylring ein- oder zweifach substituiertes Benzyl, wobei die Substituenten des Phenylrings aus der Gruppe ausgewählt sein können, die aus Niederalkyl, Niederalkoxy, Nitro und Halogen besteht, oder unsubstituiertes oder durch Niederalkyl oder Halogen einfach substituiertes Benzolsulfonyl ist oder worin Y eine Gruppe der Formel -(C=O)- (Ie) oder eine Gruppe der Formel -(C=S)- (If) ist und R₁₀ Halogen, Niederalkyl, Halogenniederalkyl, Niederalkoxy, Niederalkenyloxy, Phenylniederalkenyloxy, Halogenniederalkoxy, Niederalkylthio oder jeweils unsubstituiertes oder an dem Phenylring ein- oder zweifach substituiertes Phenyl, Phenyloxy, Phenylthio, Benzyl, Benzyloxy oder Benzylthio ist, wobei die Substituenten des Phenylrings jeweils aus der Gruppe ausgewählt sein können, die aus Niederalkyl, Niederalkoxy, Nitro und Halogen besteht, mit der Massgabe, dass in einer Verbindung I, worin R₁ eine Gruppe Ib, worin R₇, R₈ und R₉ jeweils unsubstituiertes Phenyl bedeuten und X⁻ das Bromidion ist, ist, M Methylen ist, R₂ Wasserstoff ist und R₄ Methyl ist, R₃ von Wasserstoff verschieden ist, und mit der weiteren Massgabe, dass in einer Verbindung 1, worin R₁ Hydroxy ist, M Methylen ist, R₂ Wasserstoff ist und Methyl R₄ oder Ethyl ist, R₃ von Wasserstoff verschieden ist, in freier Form oder in Salzform, können zur Herstellung von Verbindungen der Formel 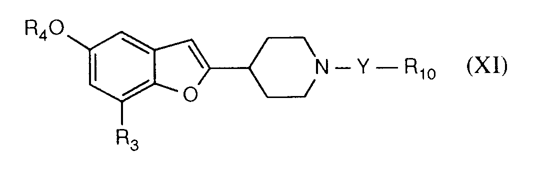 worin R₃ Halogen ist, R₄ Niederalkyl ist Y für eine Gruppe -(C=O)- (Ie) und R₁₀ für eine Gruppe X₆ steht, verwendet werden. Diese sind wertvolle Zwischenprodukte für die Herstellung entsprechender antidepressiv wirksamer Verbindungen der Formel 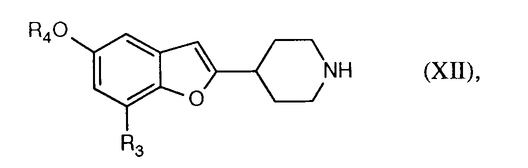 insbesondere von Brofaromin (R₃= Br; R₄= CH₃), verwendet werden, in die sie in bekannter Weise durch Abspaltung der Gruppe der Formel -Y-R₁₀ überführt werden können.

Das übliche Verfahren gemäss DE-A-2653147 zur Herstellung von Brofaromin besteht in einer aufwendigen, 8-stufigen Synthese entsprechend dem nachfolgendem Reaktionsschema A1-A8 und geht aus von 5-Methoxysalicylaldehyd, der vorab durch Formylierung von 4-Methoxyphenol nach Reimer-Tiemann (A1) hergestellt werden muss und anschliessend bromiert (A2) wird. Der erhaltene 3-Brom-5-methoxy-salicylaldehyd wird dann mit 4-Chlormethylpyridin kondensiert (A3). Das erhaltene 4-(7-Brom-5-methoxy-benzofuran-2-yl)pyridin wird zum N-Methylpyridiniumsalz N-methyliert (A4), mittels Natriumboranat zum N-Methyl-1,2,5,6-tetrahydropyridin reduziert (A5) und zum 1-Methyl-4-(7-Brom-5-methoxy-benzofuran-2-yl)piperidin hydriert (A6). Dieses wird sodann durch Umsetzung mit einem Halogenameisensäureester unter Bildung des entsprechenden 4-(7-Brom-5-methoxy-benzofuran-2-yl)piperidin-1-carbonsäureester (A7) entmethyliert, der schliesslich zu Brofaromin gespalten wird (A8).

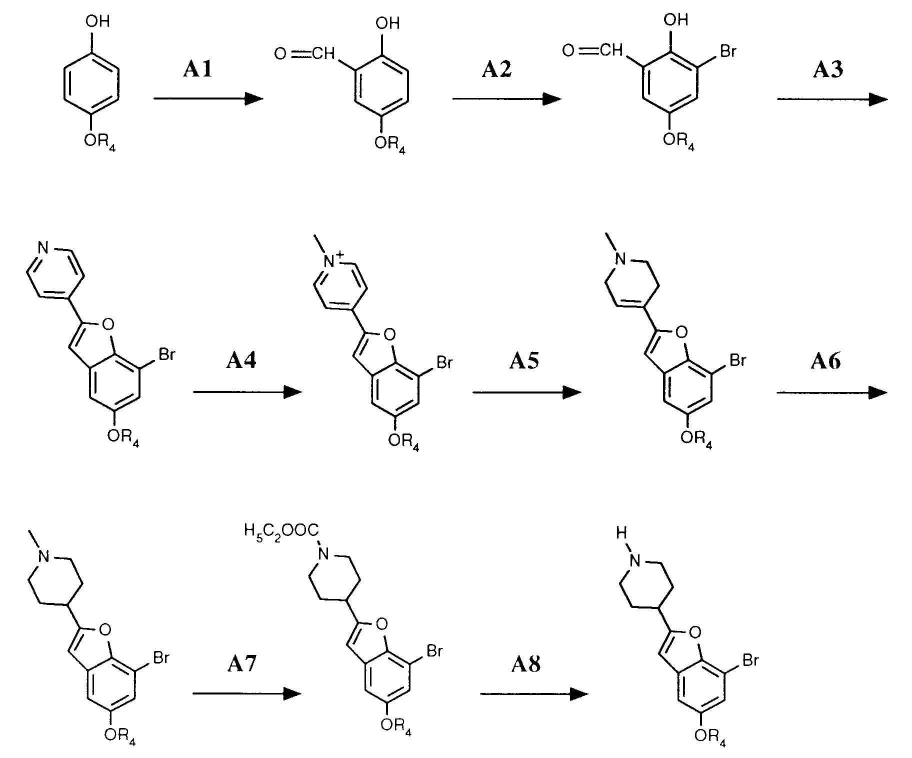

Dieses Verfahrens hat entschiedene Nachteile. Insbesondere wird ausgehend von 4-Methoxyphenol nur eine Gesamtausbeute an Brofaromin von maximal 12.7 % d.Th. erzielt. Verlustreich und vefahrenstechnisch schlecht beherrschbar sind die zum 3-Brom-5-methoxy-salicylaldehyd führenden Stufen A1 und A2 (Ausbeute 45% d.Th.). Auch die Überführung desselben in Brofaromin (A3-A8) ist auf Grund der vielen Verfahrensstufen und der nachteiligen Tatsache, dass auf der Stufe A6 bis zu 10% eines unerwünschten Desbromierungsproduktes erhalten wird, das mühsam durch mehrmalige Umkristallisation entfernt werden muss, aufwendig und verlustreich (Ausbeute 27,4 % d.Th.). Dies ist vor allem darauf zurückzuführen, dass die Ausbeute des Cyclokondensationsschrittes A3 nicht über 45 % d.Th. gesteigert werden kann. Auch bei der nachfolgenden Hydrierung von 1-Methyl-4-(7-Brom-5-methoxy-benzofuran-2-yl)-1,2,5,6-tetrahydro-pyridin und anschliessenden Entmethylierung von 1-Methyl-4-(7-Brom-5-methoxy-benzofuran-2-yl)piperidin (A6-A8) wird nur eine Produktausbeute von 64 % d.Th. erzielt.

Der Erfindung lag deshalb die Aufgabe zugrunde, ein von 4-R₄O-phenol ausgehendes Verfahren zur Herstellung von Verbindungen der Formel XII zu entwickeln, welches die Nachteile des bekannten Verfahren ganz oder teilweise vermeidet.

Diese Aufgabe wird durch das erfindungsgemässe Verfahren gelöst, welches einen in wichtigen Stufen oder in seiner Gesamtheit neuartigen und ökologisch sowie wirtschaftlich vorteilhaften Weg zu zu Verbindungen der Formel XII, insbesondere von Brofaromin, eröffnet.

Ein als allgemein anwendbar bezeichnetes Verfahren zur Herstellung von 2-furyl- bzw. 2-thienylsubstituierten Benzufuranen durch intramolekulare Wittig-Reaktion von Phophoniumverbindungen war bereits bekannt und ist bsw. in J. Med. Chem.- Chim. Ther. 18, 431-436 (1982) beschrieben. Auch einige in dem nun vorgeschlagen Verfahren zu verwendende Zwischenprodukte waren im Stand der Technik bereits bekannt. so ist in J. Med. Che. 23, 1306-1310 (1980) die Verbindung der Formel XV, worin X₅ Chlor und X₆ Benzyloxy bedeutet, und in J. Heterocycl. Chem. 27, 1047-1051 (1990) die Verbindung der Formel XIII, worin R₄ Methoxy und X⁻ Bromid ist und R₇, R₈ und R₉ Phenyl bedeuten, beschrieben.

In dem erfindungsgemäss vorgeschlagenen Verfahren kann bereits ausgehend von 5-Bromsalicylaldehyd durch Kombination der Verfahrensschritte b) c)+d) und f) und anschliessende Abspaltung der Gruppe -Y-R₁₀, nachstehend für Brofaromin (R₄= Methyl) im Schema B1-B5 gezeigt, die Produktausbeute von 27,4 % d.Th. auf 62 % d.Th. (B1 = 91 % d.Th.; B2= 89 % d.Th.; B3+B4= 86 % d.Th.; B5= 89 % d.Th.) mehr als verdoppelt werden.

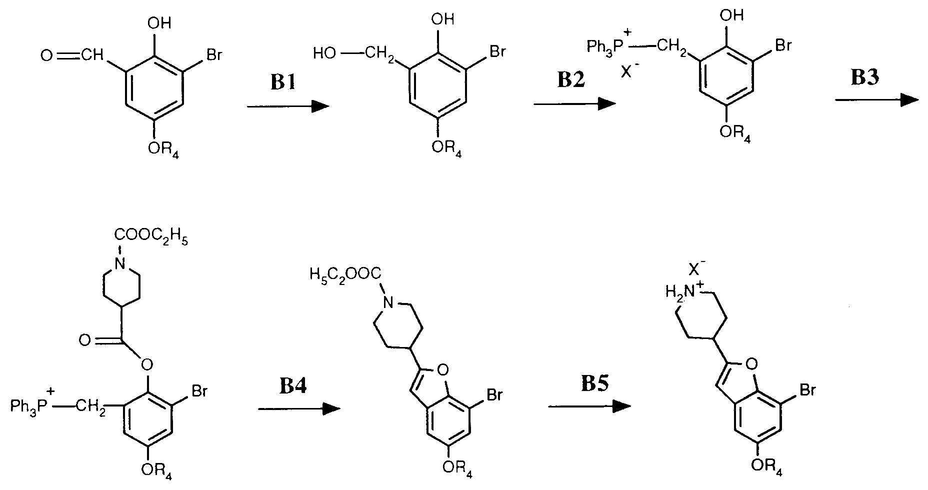

Insbesondere aber eröffnet das erfindungsgemässe Verfahren die Möglichkeit durch Kombination der Verfahrensschritte b), c)+d) und e) gemäss dem Schema C1-C3 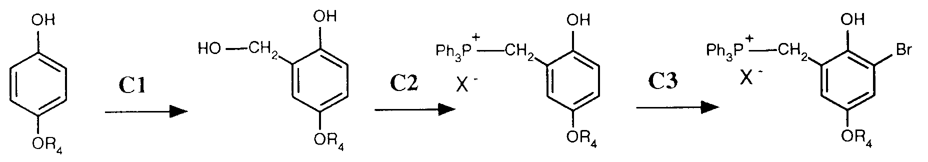 oder durch Kombination der Verfahrensschritte a), b) und c)+d) gemäss dem Schema D1-D3 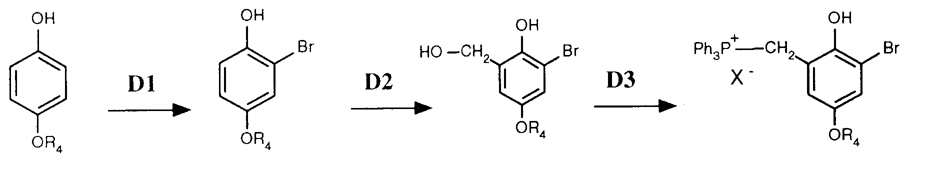 oder durch Kombination der Verfahrensschritte a), b) und c)+d) gemäss dem Schema E1-E4 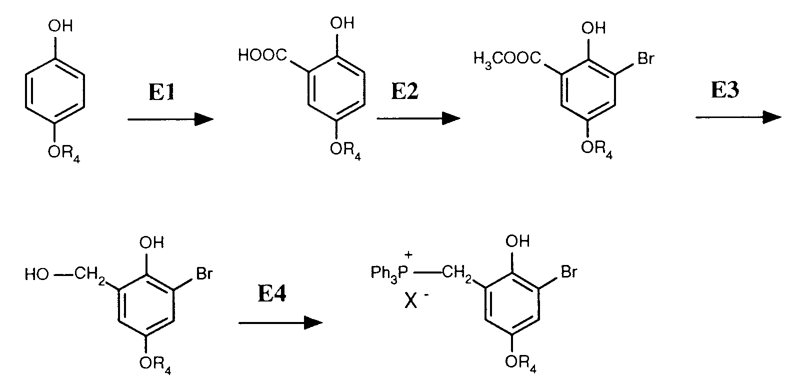 bei der Herstellung des (3-Brom-2-hydroxy-5-methoxybenzyl)phosphoniumhalogenides den verlustreichen Umweg über die Aldehydstufen zu umgehen und so die Ausbeute von 34,5 % d.Th. nochmals auf 37,9 % d.Th. (C1-C3), 41.8 % d.Th. (D1-D3) bzw. 50,2 % d.Th. (E1-E4) zu erhöhen.

Ausgehend von 4-Methoxyphenol können erfindungsgemäss leicht folgende Ausbeuten an Brofaromin-hydrochlorid erzielt werden:
Stufen B1-B5: 27.8 %;
Stufen C1-C3, B3-B5: 29.0 %;
Stufen D1-D3, B3-B5: 32.0 % und
Stufen E1-E4, B3-B5: 38.4 %.

Die Erfindung betrifft dementsprechend auch ein neuartiges Verfahren zur Herstellung von Verbindungen der Formel 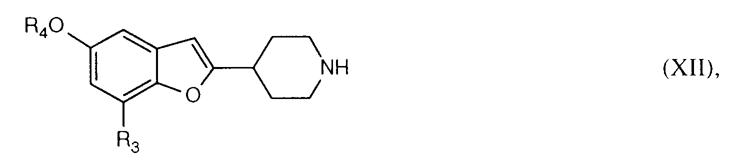 worin R₃ Halogen und R₄ Niederalkyl ist, und ihrer Salze, dadurch gekennzeichnet, dass man
g) eine Verbindung der Formel 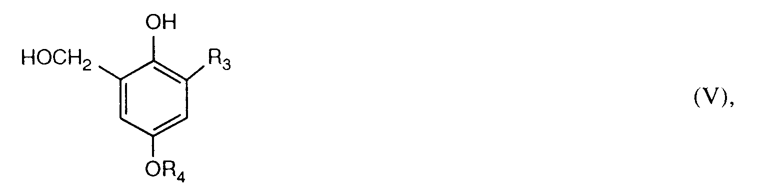 worin R₃ Halogen und R₄ Niederalkyl ist, in Gegenwart einer Halogenwasserstoffsäure der Formel HX₂, worin X₂ Halogen ist, mit einer Verbindung der Formel VIIc 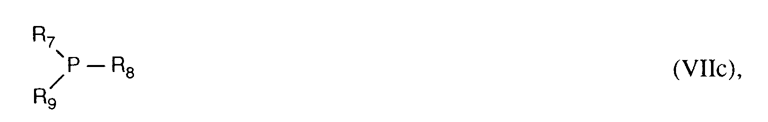 worin R₇, R₈ und R₉ jeweils unsubstituiertes Phenyl darstellen, oder mit dem Säureadditionssalz einer Verbindung der Formel VIIc und einer Halogenwasserstoffsäure der Formel HX₂, worin X₂ Halogen ist, umsetzt oder eine Verbindung der Formel 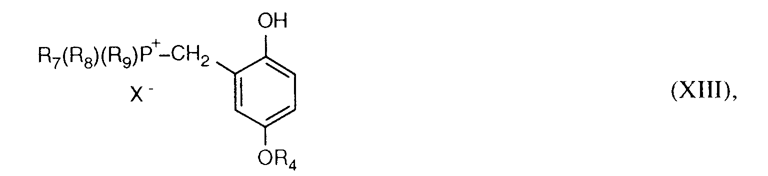 worin R₄ Niederalkyl ist und worin R₇, R₅ und R₉ jeweils unsubstituiertes Phenyl darstellen, durch Behandlung mit elementarem Halogen in einem Niederalkanol in m-Stellung zur Gruppe der Formel R₇(R₈)(R₉)P⁺CH₂- halogeniert, jeweils
h) die erhaltene Verbindung der Formel 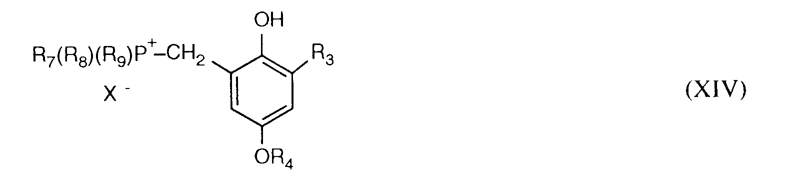 mit einer Verbindung der Formel 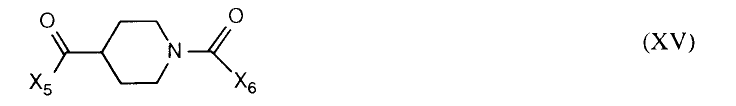 oder einem Salz davon umsetzt, worin X₅ Halogen oder eine Gruppe der Formel X₇-O- darstellt, in der X₇ Niederalkansulfonyl, Niederalkoxycarbonyl oder Niederalkoxycarbonyl ist oder eine Gruppe der Formel 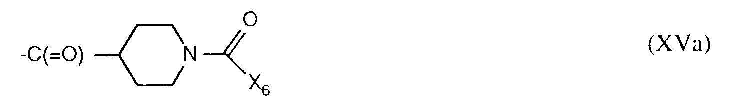 bedeutet, und X₆ für Niederalkoxy, Niederalkenyloxy oder gegebenenfalls durch Niederalkyl, Niederalkoxy, Halogen oder Nitro substituiertes Phenylniederalkoxy steht,
i) die erhaltene Verbindung der Formel 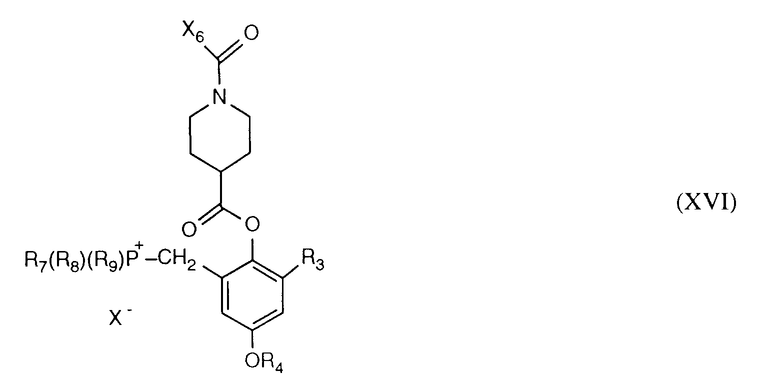 zur entsprechenden Verbindung der Formel 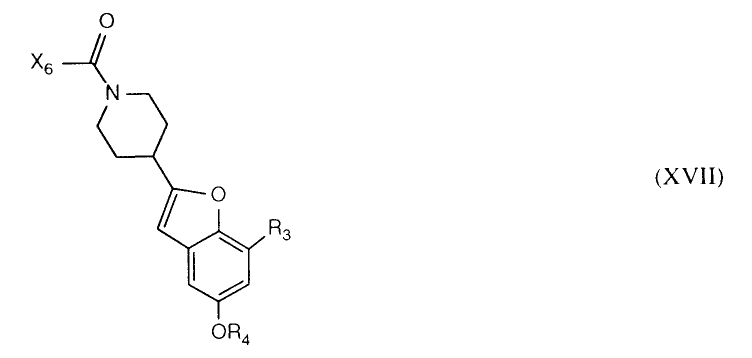 cyclisiert,
j) aus dieser die Gruppe der Formel -C(=O)-X₆ abspaltet und gewünschtenfalls eine erhaltene freie Verbindung in ein Säureadditionssalz oder ein erhaltenes Säureadditionssalz in die freie Verbindung oder in ein anderes Säureadditionssalz überführt.

Die Durchführung der verfahrensgemässen Reaktionen erfolgt in Analogie zur Reaktions- und Bildungsweise von Ausgangsstoffen bzw. Zwischenprodukten der Formeln III bis IX, insbesondere wie vorstehend unter den zu Verbindungen der Formel I führenden Verfahrensvarianten a) bis f) beschrieben. Dabei werden, auch wenn nachstehend nicht ausdrücklich erwähnt, die jeweils üblichen Hilfsmittel, wie Katalysatoren, Kondensations- sowie Solvolysemittel und/oder Lösungs- bzw. Verdünnungsmittel, und Reaktions-, wie Temperatur- und Druckbedingungen, sowie gegebenenfalls Schutzgase verwendet.

Gemäss der Verfahrensvariante g) erfolgt die Behandlung von Verbindungen der Formeln III bzw. XIII mit Halogen beispielsweise wie vorstehend unter der Verfahrensvariante a) bzw. e) beschrieben, die Reduktion von Verbindungen der Formel IV sowie die Umsetzung von Verbindungen der Formel III mit Paraformaldehyd oder Trioxan beispielsweise wie vorstehend unter der Verfahrensvariante b) beschrieben und die Umsetzung von Verbindungen der Formeln V und VIIc beispielsweise wie vorstehend unter der Verfahrensvariante d) beschrieben.

Die Umsetzung von Verbindungen der Formeln XIV und X gemäss der Verfahrensvariante h) erfolgt beispielsweise in analoger Weise wie vorstehend für die Verfahrensvariante f) angegeben.

Die Cyclisierung gemäss der Verfahrensvariante i) erfolgt beispielsweise in Gegenwart eines basischen Kondensationsmittels, wie eines Alkalimetallcarbonates, wie Kaliumcarbonat, in einem Ester oder Nitril einer Niederalkansäure, wie Acetonitril, vorteilhaft unter Ausfällen des als Nebenprodukt gebildeten Phosphoniumhalogenides durch Zugabe eines aliphatischen Kohlenwasserstoffes, wie eines C₅-C₁₀-Alkans, z.B. von Hexan, zu der Lösung des Rohproduktes in einem araliphatischen Kohlenwasserstoff, wie Toluol.

Die Abspaltung der Gruppe der Formel -C(=O)-R₁₀ gemäss der Verfahrensvariante j) kann in üblicher Weise durchgeführt werden, beispielsweise durch Basebehandlung, insbesondere mit Kaliumhydroxid in Ethylenglykol.

Die Erfindung betrifft auch diejenigen Ausführungsformen der vorstehend beschriebenen Verfahren, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Neue Ausgangsstoffe, die speziell für die Herstellung der erfindungsgemässen Verbindungen der Formel I entwickelt wurden, wie beispielsweise die neuen Verbindungen der Formel XV 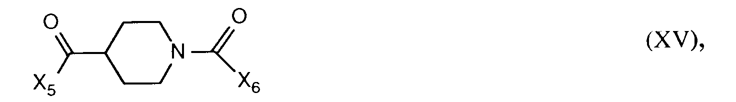 worin X₅ Halogen oder eine Gruppe der Formel X₇-O- darstellt, in der X₇ Niederalkansulfonyl, Niederalkoxycarbonyl, Niederalkanoyl oder eine Gruppe der Formel 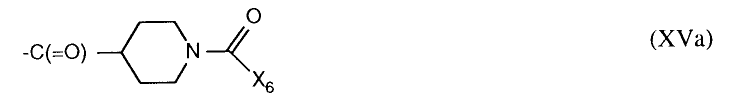 bedeutet, und X₆ für Niederalkoxy, Niederalkenyloxy oder gegebenenfalls durch Niederalkyl, Niederalkoxy, Halogen oder Nitro substituiertes Phenylniederalkoxy steht, mit der Massgabe, dass X₆ von unsubstituiertem Benzyl verschieden ist, wenn X₅ Chlor ist, und ihre Salze, die Verfahren zu ihrer Herstellung und ihre Verwendung als Zwischenprodukte bilden ebenfalls einen Gegenstand der Erfindung.

Dabei bedeutet Halogen insbesondere Chlor oder Brom, Niederalkansulfonyl beispielsweise C₁-C₄-Alkansulfonyl, wie Methan- oder Ethansulfonyl, Niederalkanoyl beispielsweise C₁-C₇-Alkanoyl, wie Acetyl, Propionyl, Butyryl oder Pivaloyl, Niederalkoxycarbonyl beispielsweise C₁-C₄-Alkoxycarbonyl, wie Methoxycarbonyl, Ethoxycarbonyl, Propyloxycarbonyl, Isopropyloxycarbonyl, Butyloxycarbonyl, Isobutyloxycarbonyl, Sekundärbutyloxycarbonyl oder Tertiärbutyloxycarbonyl, Niederalkoxy beispielsweise C₁-C₄-Alkoxy, wie Methoxy, Ethoxy, Propyloxy, Isopropyloxy, Butyloxy, Isobutyloxy, Sekundärbutyloxy oder Tertiärbutyloxy, Niederalkenyloxy insbesondere Allyloxy und Phenylniederalkoxy insbesondere Benzyloxy.

Die Erfindung betrifft beispielsweise solche Verbindungen der Formel XV, worin X₅ Halogen oder eine Gruppe der Formel X₇-O)- darstellt, in der X₇ Niederalkansulfonyl oder Niederalkoxycarbonyl bedeutet, und X₆ für Niederalkoxy steht, und ihre Salze, Verfahren zu ihrer Herstellung und ihre Verwendung als Zwischenprodukte.

Die Erfindung betrifft namentlich die in den Beispielen genannten Verbindungen der Formel XV, Verfahren zu ihrer Herstellung und ihre Verwendung als Zwischenprodukte.

Das Verfahren zur Herstellung von Verbindungen der Formel XV ist beispielsweise dadurch gekennzeichnet, dass man eine Verbindung der Formel XVIII 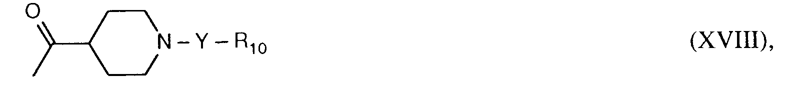 worin X₆ und R₁₀ die angegebenen Bedeutungen haben, oder ein Salz davon mit einem den Rest X₅ einführenden Mittel umsetzt und gewünschtenfalls eine erhaltene Verbindung in eine andere Verbindung der Formel XV überführt und/oder eine verfahrensgemäss erhältliche freie Verbindung in ein Salz oder ein verfahrensgemäss erhältliches Salz in die entsprechende freie Verbindung überführt.

Einen Rest X₅ einführende Mittel sind beispielsweise Halogenierungsmittel, wie Halogenide von Sauerstoffsäuren des Schwefels oder Phosphors, wie Thionylchlorid, Thionylbromid, Phosphortrichlorid oder Phosphorpentachlorid, oder Verbindungen der Formel X₇-X₅ (XIX), worin X₅ und X₇ die vorstehend angegebenen Bedeutungen haben, wie entsprechende Niederalkansulfonylhalogenide (XV; X₇= Niederalkansulfonyl, X₅= Halogen), Niederalkansäurehalogenide bzw. Niederalkansäureanhydride (XV; X₇= Niederalkanoyl, X₅= Halogen oder -OX₇) oder Halogenameisensäureniederalkylester (XV; X₇= Niederalkoxycarbonyl, X₅= Halogen).

Die Umsetzung erfolgt in üblicher Weise, beispielsweise in Gegenwart eines basischen Kondensationsmittels, wie einer tertiären organischen Stickstoffbase, wie von Pyridin oder eines aliphatischen oder cycloaliphatischen Amins, z.B. von Triethylamin, Piperidin oder N-Methylmorpholin, vorteilhaft in einen, araliphatischen oder halogenaliphatischen Kohlenwasserstoff, wie Toluol (oder Dichlormethan, und im Temperaturbereich von etwa -25° C bis etwa 50° C, beispielsweise bei etwa 0° C bis etwa 25° C.

Die nachfolgenden Beispiele dienen zur Illustration der Erfindung; Temperaturen sind in Celsiusgraden, Drucke in mbar angegeben.
Beispiel 1: Eine Suspension von 81.8 g 2-Brom-4-methoxy-phenol und 24.9 g Borsäure in 55 ml Toluol wird am Wasserabscheider unter Rückfluss erhitzt, bis sich kein Wasser mehr abscheidet (ca. 12 Stunden). Anschliessend wird die dünne hellbraune Suspension auf 90° abgekühlt und innert 40 Minuten portionsweise mit 13 g Paraformaldehyd versetzt. Man rührt das Gemisch 1 Stunde bei 90° nach, versetzt nach anschliessendem Abkühlen auf 70° mit 100 ml Wasser, kühlt danach weiter auf 20° ab und stellt mit konzentrierter Natronlauge auf pH 8.5. Die braune Suspension wird 30 Minuten gerührt und mit konzentrierter Schwefelsäure auf pH 2.5 gestellt. Der nach dem Absaugen erhaltene Filterrückstand wird zweimal mit je 50 ml Essigsäureethylester gewaschen. Die vereinigten Filtrate werden gut durchmischt und die organische Phase abgetrennt und eingedampft. Der verbleibende ölige Rückstand wird an Kieselgel mit Toluol/Essigsäureethylester (4:1) als Laufmittel chromatographiert. Der so erhältliche 3-Brom-2-hydroxy-5-methoxy-benzylalkohol besitzt einen R_{f}-Wert von 0.3; Ausbeute 50% d.Th.
Beispiel 2: Eine Lösung von 160 g 3-Brom-2-hydroxy-5-methoxy-benzaldehyd in 800 ml Ethanol wird bei 5 bis 10° innert 1 Stunde portionsweise mit 18 g Natriumborhydrid versetzt. Die erhaltene Suspension wird mit Schwefelsäure (10%) auf pH 2.5 gestellt und das Gemisch im Vakuum auf 500 ml eingeengt. Man gibt 200 ml Essigsäureethylester zu, trennt die organische Phase ab, extrahiert die wässrige Phase zweimal mit je 100 ml Essigsäureethylester, wäscht die vereinigten organischen Phasen mit Wasser, trocknet sie über Natriumsulfat und dampft sie ein. Der erhaltene ölige Rückstand wird aus Toluol kristallisiert. Man erhält so den 3-Brom-2-hydroxy-5-methoxy-benzylalkohol in Form von farblosen Kristallen [Smp.: 76°; IR (KBr): 3500, 3280, 2930, 1580, 1470, 1425, 1285, 1235, 1190, 1165, 1125, 1060, 1050, 1040 cm⁻¹; ¹H-NMR (360 MHz, CDCl₃): 2.40 (br. s, 1H, CH₂OH), 3.75 (s, 3H, OCH₃), 4.74 (s. 2H, CH₂OH), 6.34 (br. s, 1H, OH), 6.73 (d, 1H), 6.97 (d, 1H) ppm]; Ausbeute 91% d.Th.
Beispiel 3: In eine Lösung von 459 g Triphenylphosphan und 387 g 3-Brom-2-hydroxy-5-methoxy-benzylalkohol in 600 ml Essigsäureethylester werden bei 40 bis 60° innert 30 Minuten 67 g Chlorwasserstoff eingeleitet. Anschliessend wird 5 Stunden bei 75° gerührt, wobei das Produkt nach 30 Minuten zu kristallisieren beginnt. Man kühlt auf 0° ab, rührt eine weitere Stunde und saugt dann bei dieser Temperatur das Kristallisat ab. Man erhält so das [(3-Brom-2-hydroxy-5-methoxy-phenyl)methyl]-triphenyl-phosphoniumchlorid in Form von farblosen Kristallen [Smp.: 257° (Zersetzung); IR (KBr): 3010, 2880, 1605, 1585, 1565, 1440, 1420, 1330, 1260, 1225, 1150 cm-¹; ¹H-NMR (360 MHz, CH₃OH-d₄): 3.50 (s, 3H, OCH₃), 4.84 (d, 2H, CH₂P⁺), 6.49 (dd, 1H), 7.05 (dd, 1H), 7.61 bis 7.77 (m, 12H), 7.88 (m, 3H) ppm]; Ausbeute 89% d.Th.
Beispiel 4: Zu einer Lösung von 50 g [(2-Hydroxy-5-methoxy-phenyl)methyl]-triphenyl-phosphoniumbromid in 1 l Methanol werden bei 5° innert 1.5 Stunden 16 g Brom zugetropft. Man engt dann die Lösung im Vakuum bei 20° auf 130 ml ein, gibt innert 2 Stunden 250 ml Essigsäureethylester zu, rührt die entstandene gelbe Suspension 2 Stunden bei 0°, saugt die schwach gelben Kristalle ab und kristallisiert sie aus Methanol um. Man erhält so das [(3-Brom-2-hydroxy-5-methoxy-phenyl)methyl]-triphenyl-phosphonium-bromid in Form farbloser Kristalle [Smp.: 259°; IR (KBr): 2875, 1585, 1565, 1415, 1325, 1200, 1165, 1150 cm⁻¹; ¹H-NMR (360 MHz, DMSO-d₆): 3.43 (s, 3H, OCH₃), 5.03 (d, 2H, CH₂P⁺), 6.42 (dd, 1H), 7.08 (dd, 1H), 7.61 bis 7.80 (m, 12H), 7.92 (m, 3H), 9.00 (br. s, 1H, OH) ppm]; Ausbeute 76% d.Th.
Beispiel 5: In eine Lösung von 49.8 g 2-Hydroxy-5-methoxy-benzylalkohol und 115.8 g Triphenylphosphan in 150 ml Acetonitril werden bei 40 bis 60° innert 30 Minuten 18 g Chlorwasserstoff eingeleitet. Anschliessend wird 5 Stunden bei 75° gerührt, wobei das Produkt nach ca. 30 Minuten zu kristallisieren beginnt. Man kühlt auf 0° ab, rührt eine weitere Stunde und saugt dann bei dieser Temperatur das Kristallisat ab. Man erhalt so das [(2-Hydroxy-5-methoxy-phenyl)methyl]-triphenyl-phosphoniumchlorid in Form farbloser Kristalle [Smp.: 270° (Zersetzung); IR (KBr): 2990, 1585, 1395, 1295, 1240, 1145, 995 cm⁻¹; ¹H-NMR (360 MHz, CH₃OH-d₄: 3.47 (s, 3H, OCH₃), 4.74 (d, 2H, CH₂P⁺), 6.40 (dd, 1H), 6.62 (d, 1H), 6.73 (m, 1H), 7.56 bis 7.72 (m, 12H), 7.87 (m, 3H) ppm]; Ausbeute 78% d.Th.
   Zu einer Lösung von 35 g [(2-Hydroxy-5-methoxy-phenyl)methyl]-triphenyl-phosphoniumchlorid in 700 ml Methanol werden bei 5° innert 1.5 Stunden 13 g Brom zugetropft. Man engt die Lösung im Vakuum bei 20° auf 110 ml ein, gibt innert 2 Stunden 220 ml Essigsäureethylester zu, rührt die entstandene gelbe Suspension 2 Stunden bei 0°, saugt die schwach gelben Kristalle ab und kristallisiert sie aus Methanol um. Man erhält ein Gemisch von farblosen Kristallen (Smp.: 210°), das laut AgNO₃-Titer aus 22.4 Gewichtsprozent [(3-Brom-2-hydroxy-5-methoxy-phenyl)methyl]-triphenyl-phosphoniumchlorid und 77.6 Gewichtsprozent [(3-Brom-2-hydroxy-5-methoxy-phenyl)methyl]-triphenyl-phosphoniumbromid besteht; Ausbeute 76% d.Th.
Beispiel 6: Eine Lösung von 578.2 g 4-Carboxy-1-ethoxycarbonyl-piperidin in 1200 ml Toluol wird zunächst mit 1.0 g N,N-Dimethylformamid und dann bei 68 bis 70° innert 2 Stunden mit 369.0 g Thionylchlorid versetzt. Man rührt noch 30 Minuten bei 70° nach, destilliert dann im Vakuum das Toluol ab und entgast den Rückstand anschliessend ca. 30 Minuten bei Raumtemperatur im Hochvakuum. Man erhält so das 4-Chlorcarbonyl-1-ethoxycarbonyl-piperidin in Form eines schach gelben Oels [Gehalt an Produkt nach NaOH- und AgNO₃-Titer: 98%; IR (Film): 2960, 2870, 1790, 1695, 1470, 1435, 1300, 1230, 1130, 960, 765 cm⁻¹]. Das Produkt destilliert unzersetzt bei Kp= **96-98**° (0,107-0,120 mbar) [(0.08-0.09 Torr)]; Destillationsausbeute 94.7% d.Th.
Beispiel 7: In analoger Weise wie in Beispiel 6 beschrieben erhält man ausgehend von 4-Carboxy-1-allyloxycarbonyl-piperidin 4-Chlorcarbonyl-1-allyloxycarbonyl-piperidin.
Beispiel 8: In einem trockenen Sulfierkolben löst man bei 20 ° C 24.1 g (120 mmol) 4-Carboxy-1-ethoxycarbonyl-piperidin in 150 ml Toluol. Anschliessend kühlt man auf 0 ° C und dosiert in 5 Minuten 13.8 g (120 mmol) Methansulfonylchlorid zu. Danach dosiert man innerhalb von 15 Minuten bei 0 ° C eine Lösung von 12.1 g (120 mmol) N-Methylmorpholin in 50 ml Toluol zu, lässt noch weitere 30 Minuten bei 20 ° C rühren, filtriert ausgefallenes N-Methylmorpholin-Hydrochlorid unter Feuchtigkeitsausschluss ab. Die entstandene gelbliche Lösung von 1-Ethoxycarbonyl-4-methansulfonyloxycarbonyl-piperidin in Toluol kann ohne weitere Reinigung in der nächsten Stufe eingesetz werden.
Beispiel 9: In analoger Weise wie in Beispiel 8 beschrieben erhält man durch Umsetzung von 4-Carboxy-1-ethoxycarbonyl-piperidin mit Pivaloylchlorid 1-Ethoxycarbonyl-4-pivaloyloxycarbonyl-piperidin.
Beispiel 10: In einen, trockenen Sulfierkolben werden bei 20 ° C 27.7 g (138 mmol) 4-Carboxy-1-ethoxycarbonyl-piperidin in 120 ml Dichlormethan gelöst. Nach Abkühlen auf -10 ° C gibt man innerhalb von 5 Minuten eine Lösung von 18.48 g (138 mmol) Chlorameisensäureisobutylester in 15 ml Dichlormethan zu. Nach 5-minütigem Rühren und Kühlen auf - 10 ° C dosiert man innerhalb von 15 Minuten 13.96 g (138 mmol) Triäthylamin gelöst in 15 ml Dichlormethan zu. Man rührt noch weitere 30 Minuten bei 0 ° C und verwendet die entstandene klare, hellgelbe Lösung von 1-Ethoxycarbonyl-4-isobutyloxycarbonyloxycarbonyl-piperidin direkt in der nächsten Stufe.
   Um Lösungsmittelgemische in der nächsten Stufe zu vermeiden (siehe Beispiel 16) kann das Dichlormethan unter Feuchtigkeitsausschluss im Vakuum abdestilliert und der ölige Rückstand in wasserfreiem Acetonitril gelöst werden.
Beispiel 11: In analoger Weise wie in den Beispielen 6 oder 8 beschrieben erhält man durch Umsetzung von 4-Carboxy-1-ethoxycarbonyl-piperidin mit 4-Chlorcarbonyl-1-ethoxycarbonyl-piperidin 1,1'-Bis(ethoxycarbonyl)piperidin-4-carbonsäureanhydrid.
Beispiel 12: Unter Luftausschluss werden 43.9 g 4-Chlorcarbonyl-1-ethoxycarbonyl-piperidin in 250 ml wasserfreiem Acetonitril (entgast) gelöst und die Lösung mit 53.4 g [(3-Brom-2-hydroxy-5-methoxy-phenyl)methyl]-triphenyl-phosphoniumchlorid versetzt. Zu der erhaltenen Suspension werden bei 25 bis 30° innert 30 Minuten 20 g Pyridin zugetropft. Man erhitzt das Gemisch 5 Stunden zum Rückfluss, hydrolysiert es dann mit 100 ml Natriumcarbonatlösung (15%), trennt die organische Phase ab, wäscht sie nacheinander mit 100 ml 1N Salzsäure und 100 ml Wasser, dampft sie im Vakuum ein und löst den verbleibenden öligen Rückstand in 50 ml Dichlormethan. Zu dieser Lösung werden unter starkem Rühren bei 20° innert 30 Minuten 200 ml Essigsäureethylester zugetropft, wobei das Produkt auskristallisiert. Die erhaltene Kristallsuspension wird am Rotationsverdampfer unter schwachen, Vakuum auf 150 ml eingeengt und dann 2 Stunden bei 0° gerührt. Nach Absaugen und Trocknen erhält man das [(3-Brom-2-[(1-ethoxycarbonyl-piperid-4-yl)carbonyloxy]-5-methoxy-phenyl)methyl]-triphenyl-phosphoniumchlorid [Smp.: 196°; IR (KBr): 3415, 3060, 2855, 2780, 1600, 1565, 1385, 1270, 1190, 1165 cm⁻¹; ¹H-NMR (360 MHz, CDCl₃): 1.28 (t, 3H, CO₂CH₂CH₃), 1.45 (m, 2H), 1.75 (m, 2H), 2.58 (m, 1H), 2.87 (m, 2H), 3.47 (s, 3H, OCH₃), 4.00 (m, 2H), 4.13 (q, 2H, CO₂CH₂CH₃), 5.37 (br. d, 2H, CH₂P⁺), 6.87 (dd, 1H), 7.07 (dd, 1H), 7.54 bis 7.87 (m, 15H) ppm]; Ausbeute 66% d.Th.
Beispiel 13: In eine Suspension von 50 g 3-Brom-2-hydroxy-5-methoxy-benzylalkohol in 300 ml wasserfreier Essigsäure werden bei Raumtemperatur innert 30 Minuten 20 g Bromwasserstoff eingeleitet. Man rührt 5 Stunden bei Raumtemperatur nach und dampft die entstandene Lösung ein. Der schwarz-braune ölige Rückstand, welcher beim Stehenlassen bei Raumtemperatur langsam kristallisiert, wird unter Zusatz von wenig Aktivkohle aus Essigsäureethylester/Cyclohexan (5:1) kristallisiert. Man erhält so das 3-Brom-2-hydroxy-5-methoxy-benzylbromid [¹H-NMR (360 MHz, CDCl₃): 3.75 (s, 3H, OCH₃), 4.53 (s, 2H, CH₂Br), 5.45 (s, 1H, OH), 6.85 (d, 1H), 7.00 (d, 1H) ppm].
Beispiel 14: 20.0 g 3-Brom-2-hydroxy-5-methoxy-benzylbromid werden mit 8.8 g Trimethylphosphit versetzt und das Gemisch 3 Stunden unter Rühren auf 140° erwärmt (laut DC vollständiger Umsatz). Das resultierende Oel wird mit Toluol/Essigsäureethylester (4:1) als Laufmittel über wenig Kieselgel filtriert. Man erhält so den [(3-Brom-2-hydroxy-5-methoxy-phenyl)methyl]phosphonsäuredimethylester [¹H-NMR (360 MHz, CDCl₃): 3.20 (d, 2H, CH₂P, ²J_{P-H}= 21 Hz), 3.70 (s, 3H, OCH₃), 3.74 (d, 6H, 2 x OCH₃, ³J_{P-H}= 1 Hz), 6.70 (m, 1H), 7.07 (m, 1H) ppm].
Beispiel 15: In eine Suspension von 20 g gemahlenem Kaliumcarbonat (wasserfrei)in 100 ml Acetonitril werden unter Luftausschluss bei 25 bis 30° innert 30 Minuten 50 g [(3-Brom-2-[(1-ethoxycarbonylpiperid-4-yl)carbonyloxy]-5-methoxy-phenyl)methyl]-triphenyl-phosphoniumchlorid eingetragen. Das Gemisch wird 2 Stunden bei Raumtemperatur gerührt, anschliessend mit 80 ml Wasser versetzt und mit konzentrierter Salzsäure auf pH 1.0 angesäuert. Die organische Phase wird abgetrennt und eingedampft und der ölige Rückstand in 50 ml Toluol aufgenommen und bei 20° innert 1 Stunde mit 100 ml Hexan versetzt, wobei die Hauptmenge (ca. 90%; Gehalt laut HPLC: 90%) des entstandenen Triphenylphosphanoxids auskristallisiert. Die Kristallsuspension wird abgesaugt und der Filterkuchen zweimal mit je 40 ml Hexan/Toluol (3:2) gewaschen. Nach Eindampfen der vereinigten Filtrate und Chromatographie des Rückstands an Kieselgel mit Toluol/Essigsäureethylester (2:1) als Laufmittel erhält man das 4-(7-Brom-5-methoxy-benzofuran-2-yl)-1-ethoxycarbonyl-piperidin [Smp.: 85°; ¹H-NMR (360 MHz, CDCl₃): 1.28 (t, 3H, CO₂CH₂CH₃), 1.69 (m, 2H), 2.09 (m, 2H), 2.95 (m, 3H), 3.80 (s, 3H, OCH₃), 4.16 (q, 2H, CO₂CH₂CH₃), 4.14 bis 4.35 (br. m, 2H), 6.36 (s, 1H), 6.90 (d, 1H), 7.00 (d, 1H) ppm]; Ausbeute 90% d.Th.
Beispiel 16: Zu einer Suspension von 105 g [(3-Brom-2-hydroxy-5-methoxy-phenyl)-methyl]-triphenyl-phosphoniumchlorid und 115 g gemahlenem Kaliumcarbonat (wasserfrei) in 250 ml Acetonitril werden unter Luftausschluss bei 25 bis 30° innert 1.5 bis 2 Stunden 49 g 4-Chlorcarbonyl-1-ethoxycarbonyl-piperidin zugetropft. Man rührt 30 Minuten bei 25 bis 30°, versetzt anschliessend mit 350 ml Wasser und säuert mit konzentrierter Salzsäure auf pH 1.0 an. Die organische Phase wird abgetrennt und eingedampft. Der ölige Rückstand wird in 160 ml Toluol aufgenommen und innert 1 Stunde bei 20° mit 250 ml Hexan versetzt, wobei die Hauptmenge (ca. 80%; Gehalt laut HPLC: 85%) des entstandenen Triphenylphosphanoxids auskristallisiert. Die Kristallsuspension wird abgesaugt und der Filterkuchen zweimal mit je 40 ml Hexan/Toluol (3:2) gewaschen. Die vereinigten Filtrate werden eingedampft und der ölige Rückstand an Kieselgel mit Toluol/Essigsäureethylester (2:1) als Laufmittel chromatographiert. Man erhält so das 4-(7-Brom-5-methoxy-benzofuran-2-yl)-1-ethoxycarbonyl-piperidin, das mit dem gemäss Beispiel 8 erhältlichen Produkt identisch ist; Ausbeute 86% d.Th.
Beispiel 17: Zu einer Lösung von 45.9 g 2-Hydroxy-5-methoxy-benzoesäuremethylester in 125 ml Heptan werden bei 20° innerhalb von 30 bis 45 Minuten unter Rühren 43.9 g Brom zugetropft. Das Reaktionsgemisch wird sodann weitere 3 bis 4 Stunden gerührt, anschliessend mit 110 ml Wasser verdünnt, eine weitere Stunde gerührt und dann mit 295 ml Heptan und 46 ml tert.-Butylmethylether versetzt. Anschliessend erwärmt man das Gemisch auf 50 bis 55° und trennt die wässrige Phase ab. Die organische Phase wird zweimal mit je 114 ml Wasser extrahiert und auf -20° abgekühlt. Die ausgefallenen Kristalle werden abfiltriert und im Hochvakuum getrocknet. Man erhält so den 3-Brom-2-hydroxy-5-methoxy-benzoesäuremethylester; Ausbeute 82.5% d.Th.
Beispiel 18: Zu einer Lösung von 8 g Natriumborhydrid in 280 ml Tetrahydrofuran wird bei 20° innert 1 Stunde unter Rühren eine Lösung von 57 g 3-Brom-2-hydroxy-5-methoxy-benzoesäuremethylester in 110 ml Tetrahydrofuran zugetropft. Das Reaktionsgemisch wird weitere 2.5 Stunden gerührt und mit 105 ml 2N Salzsäure hydrolysiert und auf pH 2.5 eingestellt. Die wässrige Phase wird abgetrennt. Die organische Phase wird dreimal mit je 90 ml Natriumchloridlösung (10%) extrahiert, am Rotationsverdampfer eingedampft, mit 100 ml Toluol verdünnt, am Rotationsverdampfer auf 100 g eingeengt und bei 60° innert 30 Minuten zu einer auf 70 bis 80° erwärmten Suspension aus 55.1 g Triphenylphosphan, 8.8 g Chlorwasserstoff und 42 ml Toluol zugegeben. Das Reaktionsgemisch wird weitere 7 Stunden gerührt und dann auf 0 bis -5° abgekühlt. Das entstandene Kristallisat wird abfiltriert, viermal mit je 40 ml Toluol gewaschen und im Hochvakuum getrocknet. Man erhält so das [(3-Brom-2-hydroxy-5-methoxy-phenyl)methyl]-triphenyl-phosphoniumchlorid, das mit dein gemäss Beispiel 3 erhältlichen Produkt identisch ist; Ausbeute 79% d.Th.
Beispiel 19: 7.6 g 4-(7-Brom-5-methoxy-benzofuran-2-yl)-1-ethoxycarbonyl-piperidin werden in 80 ml Ethylenglykol gelöst. Nach Zugabe von 19.4 g 86%-igem Kaliumhydroxid wird die entstandene, trübe Lösung unter starkem Rühren 18 Stunden auf 160° erhitzt. Das Reaktionsgemisch wird auf 100° abgekühlt, mit 80 ml Toluol verdünnt und auf 20° weitergekühlt. Die Reaktionslösung wird zweimal mit je 1000 ml Wasser und dann viermal mit je 200 ml einer 10%-igen Lösung von Methansulfonsäure in Wasser extrahiert. Die methansulfonsaure Lösung wird durch Zugebe von 30%-iger Natronlauge auf PH 12 gestellt und mit 1000 ml Chloroform ausgeschüttelt. die Chloroformlösung wird über Natriumsulfat getrocknet, filtiert und eingedampft. Nach Kristallisieren aus Ethylacetat erhält man 4-(7-Brom-5-methoxy-benzofuran-2-yl)-piperidin vom Smp. 149-152°. Aus der Base wird durch Behandeln mit methanolischer Salzsäure und Umkristallisieren aus Methanol/Ether wird das 4-(7-Brom-5-methoxy-benzofuran-2-yl)-piperidin-hydrochlorid vom Smp. 242-243° erhalten.

## Patentansprüche

1. Eine Verbindung der Formel I 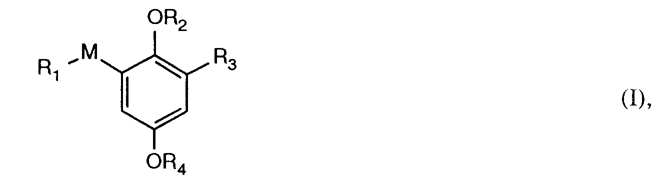 worin R₁ Hydroxy, Halogen, eine Gruppe der Formel -P(=O)(R₅)R₆ (Ia) oder eine Gruppe der Formel -P⁺(R₇)(R₈)R₉ X⁻ (Ib) ist, M Methylen ist, R₂ Wasserstoff oder eine Gruppe der Formel 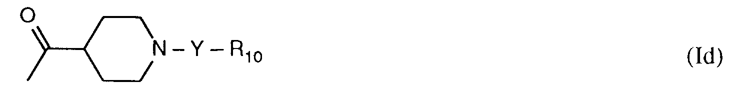 ist, R₃ Wasserstoff oder Halogen ist, R₄ Niederalkyl ist, R₅ und R₆ jeweils unabhängig voneinander Niederalkyl, Niederalkoxy, N,N-Diniederalkylamino oder jeweils unsubstituiertes oder an dem Phenylring ein- oder zweifach substituiertes Benzyl, Benzyloxy, Phenyl oder Phenyloxy darstellen, wobei die Substituenten des Phenylrings jeweils aus der Gruppe ausgewählt sein können, die aus Niederalkyl, Niederalkoxy, Nitro und Halogen besteht, R₇, R₈ und R₉ jeweils unabhängig voneinander Niederalkyl, unsubstituiertes oder ein- oder zweifach substituiertes Phenyl, wobei die Substituenten des Phenylrings jeweils aus der Gruppe ausgewählt sein können, die aus Niederalkyl, Niederalkoxy, Nitro und Halogen besteht, oder Furyl darstellen, X⁻ das Anion einer Halogenwasserstoffsäure, einer Niederalkansulfonsäure, einer Halogenniederalkansulfonsäure oder einer unsubstituierten oder durch Niederalkyl oder Halogen einfach substituierten Benzolsulfonsäure ist und worin entweder Y für eine direkte Bindung steht und R₁₀ Niederalkyl, Allyl, Cyano, Niederalkansulfonyl, Halogenniederalkansulfonyl, unsubstituiertes oder an dem Phenylring ein- oder zweifach substituiertes Benzyl, wobei die Substituenten des Phenylrings aus der Gruppe ausgewählt sein können, die aus Niederalkyl, Niederalkoxy, Nitro und Halogen besteht, oder unsubstituiertes oder durch Niederalkyl oder Halogen einfach substituiertes Benzolsulfonyl ist oder worin Y eine Gruppe der Formel -(C=O)- (Ie) oder eine Gruppe der Formel -(C=S)- (If) ist und R₁₀ Halogen, Niederalkyl, Halogenniederalkyl, Niederalkoxy, Niederalkenyloxy, Phenylniederalkenyloxy, Halogenniederalkoxy, Niederalkylthio oder jeweils unsubstituiertes oder an dem Phenylring ein- oder zweifach substituiertes Phenyl, Phenyloxy, Phenylthio, Benzyl, Benzyloxy oder Benzylthio ist, wobei die Substituenten des Phenylrings jeweils aus der Gruppe ausgewählt sein können, die aus Niederalkyl, Niederalkoxy, Nitro und Halogen besteht, mit der Massgabe, dass in einer Verbindung I, worin R₁ eine Gruppe Ib, worin R₇, R₈ und R₉ jeweils unsubstituiertes Phenyl bedeuten und X⁻ das Bromidion ist, R₂ Wasserstoff ist und R₄ Methyl ist, R₃ von Wasserstoff verschieden ist, und mit der weiteren Massgabe, dass in einer Verbindung I, worin R₁ Hydroxy ist, R₂ Wasserstoff ist und R₄ Methyl oder Ethyl ist, R₃ von Wasserstoff verschieden ist, in freier Form oder in Salzform.

2. Eine Verbindung gemäss Anspruch 1 der Formel I, worin R₁ Hydroxy, Halogen, eine Gruppe Ia oder eine Gruppe Ib ist, M Methylen ist, R₂ Wasserstoff oder eine Gruppe Id ist, R₃ Wasserstoff oder Halogen ist und R₄ Niederalkyl ist, R₅ und R₆ jeweils unabhängig voneinander Niederalkoxy darstellen, R₇, R₈ und R₉ jeweils unsubstituiertes Phenyl oder Furyl darstellen, X⁻ das Anion einer Halogenwasserstoffsäure ist, Y eine Gruppe Ie ist und R₁₀ Niederalkoxy oder Niederalkenyloxy ist, in freier Form oder in Salzform.

3. Eine Verbindung gemäss Anspruch 1 der Formel I, worin R₁ Hydroxy oder eine Gruppe Ib ist, M Methylen ist, R₂ Wasserstoff oder eine Gruppe Id ist, R₃ Halogen ist, R₄ C₁-C₄-Alkyl ist, R₇, R₈ und R₉ unsubstituiertes Phenyl darstellen, X⁻ das Anion einer Halogenwasserstoffsäure ist, Y eine Gruppe Ie ist und R₁₀ C₁-C₄-Alkoxy ist, in freier Form oder in Salzform.

4. Eine Verbindung gemäss Anspruch 1 der Formel I, worin R₁ eine Gruppe Ib ist, M Methylen ist, R₂ Wasserstoff oder eine Gruppe Id ist, R₃ Brom ist, R₄ Methyl ist, R₇, R₈ und R₉ jeweils unsubstituiertes Phenyl darstellen, X⁻ das Chloridion oder das Bromidion ist, Y eine Gruppe Ie ist und R₁₀ Ethoxy ist, in freier Form oder in Salzform.

5. 3-Brom-2-hydroxy-5-methoxy-benzylalkohol gemäss Anspruch 1.

6. [(3-Brom-2-hydroxy-5-methoxy-phenyl)methyl]-triphenyl-phosphoniumchlorid gemäss Anspruch 1.

7. [(3-Brom-2-hydroxy-5-methoxy-phenyl)methyl]-triphenyl-phosphoniumbromid gemäss Anspruch 1.

8. [(3-Brom-2-[(1-ethoxycarbonylpiperid-4-yl)carbonyloxy]-5-methoxy-phenyl)methyl]-triphenyl-phosphoniumchlorid gemäss Anspruch 1.

9. 3-Brom-2-hydroxy-5-methoxy-benzylbromid gemäss Anspruch 1.

10. [(3-Brom-2-hydroxy-5-methoxy-phenyl)methyl]phosphonsäuredimethylester gemäss Anspruch 1.

11. 3-Brom-2-hydroxy-5-methoxy-benzoesäuremethylester gemäss Anspruch 1.

12. Verfahren zur Herstellung von Verbindungen der Formel I gemäss Anspruch 1, in freier Form oder in Salzform, dadurch gekennzeichnet, dass man
a) zur Herstellung einer Verbindung der Formel I, worin R₁ Niederalkoxy ist, M zusätzlich Carbonyl bedeutet, R₂ Wasserstoff, R₃ Halogen und R₄ Niederalkyl ist, oder eines Salzes davon eine Verbindung der Formel II 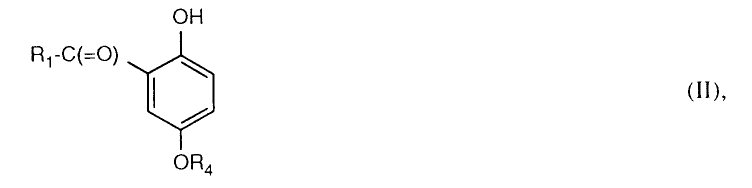 worin R₁ Hydroxy oder Niederalkoxy und R₄ Niederalkyl bedeutet, oder ein Salz davon durch Behandlung mit elementarem Halogen in einem Niederalkanol in m-Stellung zur R₁-C(=O)-Gruppe halogeniert, wobei Carboxy R₁-C(=O)-, sofern vorhanden, zu Niederalkoxycarbonyl verestert wird und/oder
b) zur Herstellung einer Verbindung der Formel I, worin R₁ Hydroxy, M Methylen, R₂ Wasserstoff, R₃ Wasserstoff oder Halogen und R₄ Niederalkyl ist, mit der Massgabe, dass R₃ von Wasserstoff verschieden ist, wenn R₄ Methyl oder Ethyl darstellt, oder eines Salzes davon eine Verbindung der Formel III 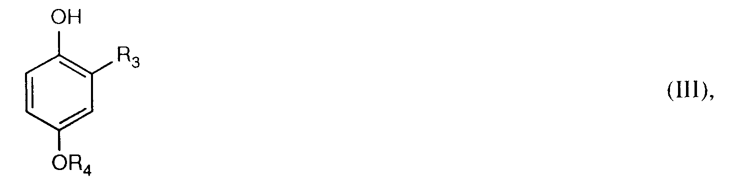 worin R₃ Wasserstoff oder Halogen und R₄ Niederalkyl ist, oder ein Salz davon mit Paraformaldehyd oder Trioxan umsetzt oder in einer Verbindung der Formel 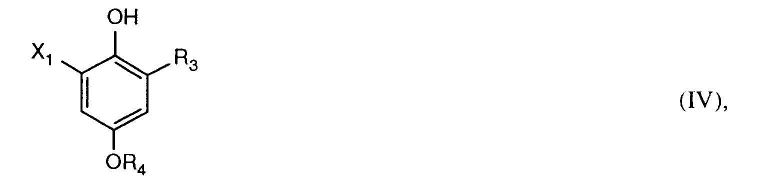 worin X₁ die Formylgruppe, Carboxy oder Niederalkoxycarbonyl bedeutet, R₃ Halogen und R₄ Niederalkyl ist, oder einem Salz davon die Gruppe X₁ zu Hydroxymethyl reduziert und/oder
c) zur Herstellung einer Verbindung der Formel I, worin R₁ Halogen, M Methylen, R₂ Wasserstoff und R₃ Halogen ist, oder eines Salzes davon in einer Verbindung der Formel 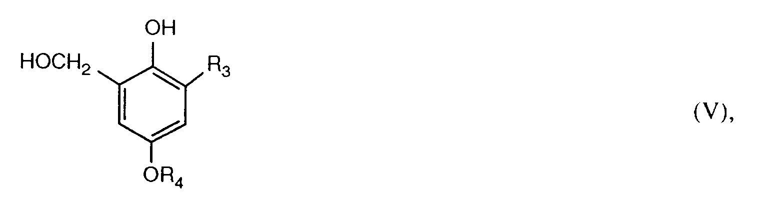 die Hydroxymethylgruppe in Halogenmethyl überführt oder eine Verbindung der Formel 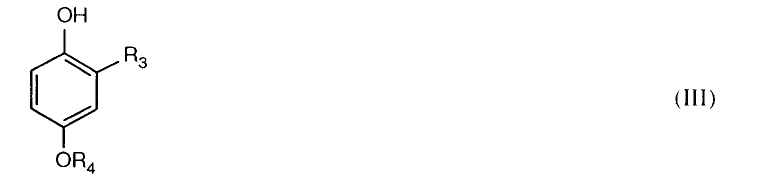 mit Parafomaldehyd oder Trioxan und einer Halogenwasserstoffsäure umsetzt und/oder
d) zur Herstellung einer Verbindung der Formel I, worin R₁ eine eine Gruppe der Formel -P(=O)(R₅)R₆ (Ia) oder eine Gruppe der Formel -P⁺(R₇)(R₈)R₉ X⁻ (Ib), M Methylen, R₂ Wasserstoff, R₃ Halogen und R₄ Niederalkyl ist und R₅, R₆, R₇, R₈, R₉ und X⁻ die unter der Formel I angegebenen Bedeutungen haben, oder eines Salzes davon eine Verbindung der Formel 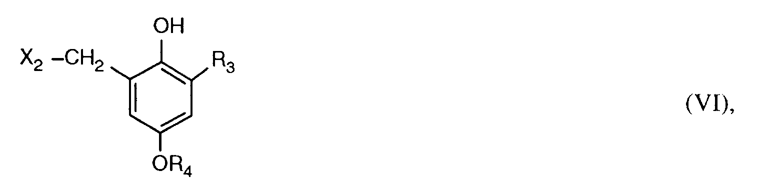 worin X₂ Halogen, R₃ Halogen und R₄ Niederalkyl ist, mit einer Verbindung der Formeln VIIa, VIIb oder VIIc 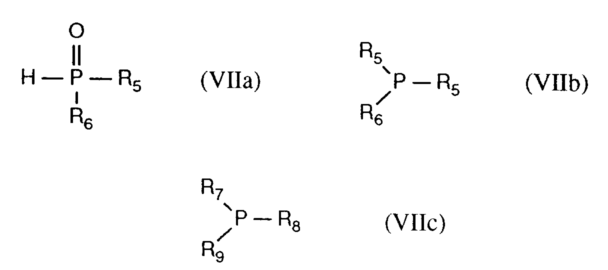 oder einem Salz davon umsetzt, worin R₅, R₆, R₇, R₈ und R₉ die unter der Formel I angegebenen Bedeutungen haben oder eine Verbindung der Formel 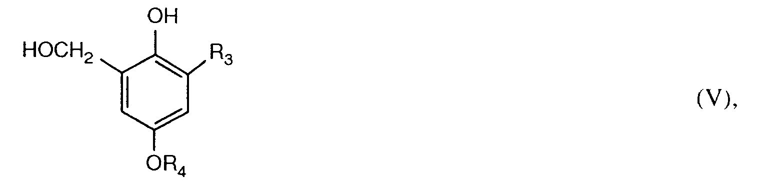 worin R₃ Halogen und R₄ Niederalkyl ist, mit einer Verbindung der Formel VIIc 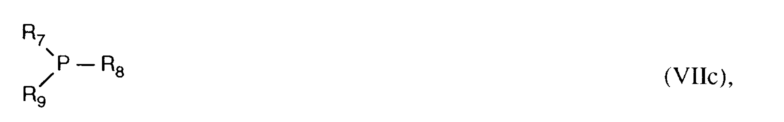 worin R₇, R₈ und R₉ die unter der Formel I angegebenen Bedeutungen haben, und einer Halogenwasserstoffsäure der Formel HX umsetzt und/oder
e) zur Herstellung einer Verbindung der Formel I, worin R, eine Gruppe der Formel -P(=O)(R₅)R₆ (Ia) oder eine Gruppe der Formel -P⁺(R₇)(R₈)R₉ X⁻ (Ib), M Methylen, R₂ Wasserstoff, R₃ Halogen und R₄ Niederalkyl ist und R₅, R₆, R₇, R₈, R₉ und X⁻ die unter der Formel I angegebenen Bedeutungen haben, oder eines Salzes davon eine Verbindung der Formel 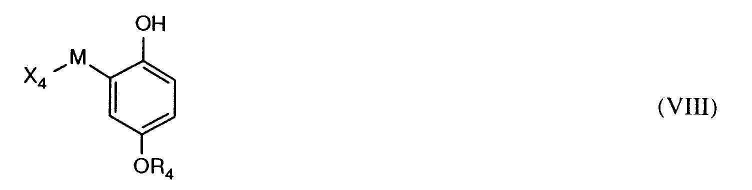 worin X₄ eine Gruppe der Formel -P(=O)(R₅)R₆ (Ia) oder eine Gruppe der Formel -P⁺(R₇)(R₈)R₉ X⁻ (Ib), M Methylen und R₄ Niederalkyl ist, in m-Stellung zur X₄-M-Gruppe halogeniert und/oder
f) zur Herstellung einer Verbindung der Formel I, worin R₁ eine Gruppe der Formel -P⁺(R₇)(R₈)R₉ X⁻ (Ib), M Methylen, R₂ eine Gruppe der Formel 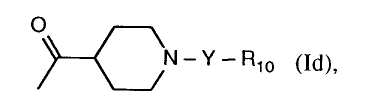 R₃ Halogen und R₄ Niederalkyl ist und R₇, R₈, R₉, R₁₀, Y und X⁻ die unter der Formel I angegebenen Bedeutungen haben, oder eines Salzes davon eine Verbindung der Formel 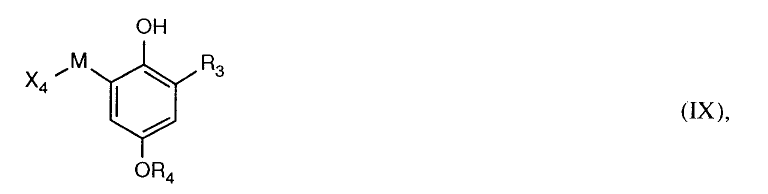 worin X₄ eine Gruppe der Formel -P⁺(R₇)(R₈)R₉ X⁻ (Ib), M Methylen, R₃ Halogen und R₄ Niederalkyl ist, und R₇, R₈, R₉ und X⁻ die unter der Formel I angegebenen Bedeutungen haben, mit einer Verbindung der Formel 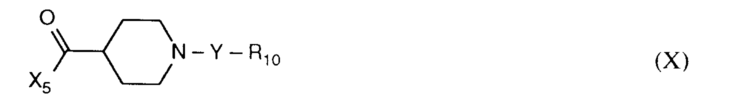 oder einem Salz davon umsetzt, worin X₅ Halogen oder eine Gruppe der Formel X₇-O- darstellt, in der X₇ Niederalkansulfonyl, Niederalkoxycarbonyl, Niederalkanoyl oder eine Gruppe der Formel 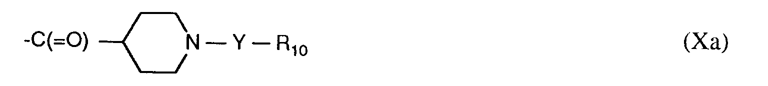 bedeutet, und R₁₀ und Y die unter der Formel I angegebenen Bedeutungen haben und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

13. Verwendung von Verbindungen der Formel I 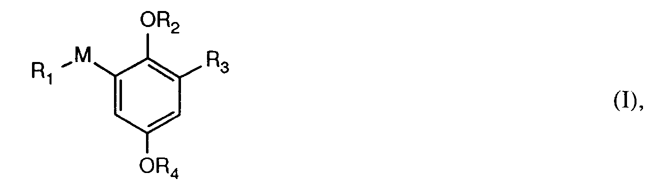 worin R₁ Hydroxy, Halogen, eine Gruppe der Formel -P(=O)(R₅)R₆ (Ia) oder eine Gruppe der Formel -P⁺(R₇)(R₈)R₉ X⁻ (Ib) ist, M Methylen ist, R₂ Wasserstoff oder eine Gruppe der Formel 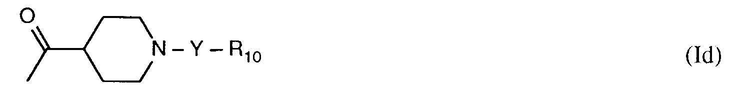 ist, R₃ Wasserstoff oder Halogen ist, R₄ Niederalkyl ist R₅ und R₆ jeweils unabhängig voneinander Niederalkyl, Niederalkoxy, N,N-Diniederalkylamino oder jeweils unsubstituiertes oder an dem Phenylring ein- oder zweifach substituiertes Benzyl, Benzyloxy, Phenyl oder Phenyloxy darstellen, wobei die Substituenten des Phenylrings jeweils aus der Gruppe ausgewählt sein können, die aus Niederalkyl, Niederalkoxy, Nitro und Halogen besteht, R₇, R₈ und R₉ jeweils unabhängig voneinander Niederalkyl, unsubstituiertes oder ein- oder zweifach substituiertes Phenyl, wobei die Substituenten des Phenylrings jeweils aus der Gruppe ausgewählt sein können, die aus Niederalkyl, Niederalkoxy, Nitro und Halogen besteht, oder Furyl darstellen, X⁻ das Anion einer Halogenwasserstoffsäure, einer Niederalkansulfonsäure, einer Halogenniederalkansulfonsäure oder einer unsubstituierten oder durch Niederalkyl oder Halogen einfach substituierten Benzolsulfonsäure ist und worin entweder Y für eine direkte Bindung steht und R₁₀ Niederalkyl, Allyl, Cyano, Niederalkansulfonyl, Halogenniederalkansulfonyl, unsubstituiertes oder an dem Phenylring ein- oder zweifach substituiertes Benzyl, wobei die Substituenten des Phenylrings aus der Gruppe ausgewählt sein können, die aus Niederalkyl, Niederalkoxy, Nitro und Halogen besteht, oder unsubstituiertes oder durch Niederalkyl oder Halogen einfach substituiertes Benzolsulfonyl ist oder worin Y eine Gruppe der Formel -(C=O)- (Ie) oder eine Gruppe der Formel -(C=S)- (If) ist und R₁₀ Halogen, Niederalkyl, Halogenniederalkyl, Niederalkoxy, Niederalkenyloxy, Phenylniederalkenyloxy, Halogenniederalkoxy, Niederalkylthio oder jeweils unsubstituiertes oder an dem Phenylring ein- oder zweifach substituiertes Phenyl, Phenyloxy, Phenylthio, Benzyl, Benzyloxy oder Benzylthio ist, wobei die Substituenten des Phenylrings jeweils aus der Gruppe ausgewählt sein können, die aus Niederalkyl, Niederalkoxy, Nitro und Halogen besteht, mit der Massgabe, dass in einer Verbindung I, worin R₁ eine Gruppe Ib, worin R₇, R₈ und R₉ jeweils unsubstituiertes Phenyl bedeuten und X⁻ das Bromidion ist, ist, M Methylen ist, R₂ Wasserstoff ist und R₄ Methyl ist, R₃ von Wasserstoff verschieden ist, und mit der weiteren Massgabe, dass in einer Verbindung I, worin R₁ Hydroxy ist, M Methylen ist, R₂ Wasserstoff ist und R₄ Methyl oder Ethyl ist, R₃ von Wasserstoff verschieden ist, in freier Form oder in Salzform zur Herstellung von Verbindungen der Formel 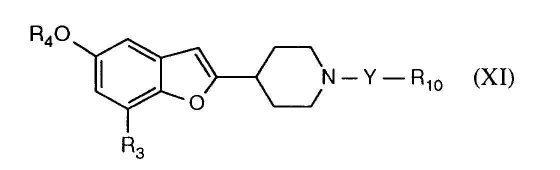 oder von Verbindungen der Formel 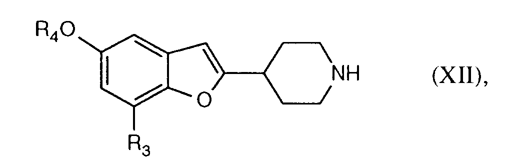 worin R₃ Halogen ist, R₄ Niederalkyl ist und R₁₀ und Y die angegebenen Bedeutungen haben, und ihrer Salze.

14. Verbindungen der Formel XV 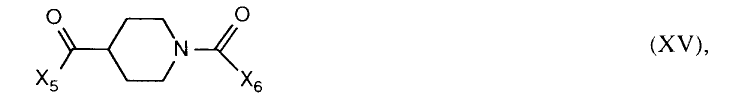 worin X₅ Halogen oder eine Gruppe der Formel X₇-O- darstellt, in der X₇ Niederalkansulfonyl, Niederalkoxycarbonyl, Niederalkanoyl oder eine Gruppe der Formel 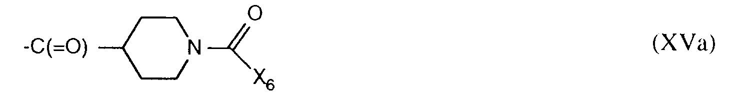 bedeutet, und X₆ für Niederalkoxy, Niederalkenyloxy oder gegebenenfalls durch Niederalkyl, Niederalkoxy, Halogen oder Nitro substituiertes Phenylniederalkoxy steht, mit der Massgabe, dass X₆ von unsubstituiertem Benzyl verschieden ist, wenn X₅ Chlor ist, und ihre Salze.

15. Verbindungen gemäss Anspruch 14 der Formel XV, worin X₅ Halogen bedeutet und X₆ für Niederalkoxy steht, und ihre Salze.

16. 4-Chlorcarbonyl-1-ethoxycarbonyl-piperidin gemäss Anspruch 14.

17. 4-Chlorcarbonyl-1-allyloxycarbonyl-piperidin gemäss Anspruch 14.

18. 1-Ethoxycarbonyl-4-methansulfonyloxycarbonyl-piperidin gemäss Anspruch 14.

19. 1-Ethoxycarbonyl-4-isobutyloxycarbonyloxycarbonyl-piperidin gemäss Anspruch 14.

20. 1-Ethoxycarbonyl-4-pivaloyloxycarbonyl-piperidin gemäss Anspruch 14.

21. 1,1'-Bis(ethoxycarbonyl)piperidin-4-carbonsäureanhydrid gemäss Anspruch 14.

22. Verfahren zur Herstellung von Verbindungen der Formel XV 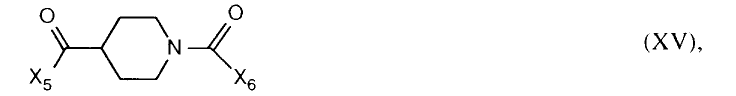 worin X₅ Halogen oder eine Gruppe der Formel X₇-O- darstellt, in der X₇ Niederalkansulfonyl, Niederalkoxycarbonyl, Niederalkanoyl oder eine Gruppe der Formel 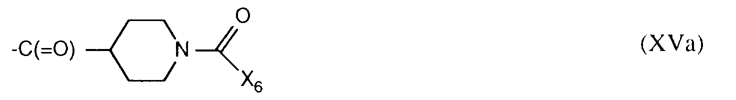 bedeutet, und X₆ für Niederalkoxy, Niederalkenyloxy oder gegebenenfalls durch Niederalkyl, Niederalkoxy, Halogen und/oder Nitro substituiertes Phenylniederalkoxy steht, mit der Massgabe, dass X₆ von unsubstituiertem Benzyl verschieden ist, wenn X₅ Chlor ist, und ihrer Salze, dadurch gekennzeichnet, dass man eine Verbindung der Formel XVIII 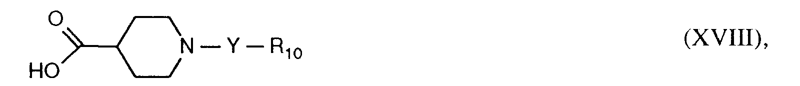 worin Y und R₁₀ die angegebenen Bedeutungen haben, oder ein Salz davon mit einem den Rest X₅ einführenden Mittel umsetzt und gewünschtenfalls eine erhaltene Verbindung in eine andere Verbindung der Formel XV überführt und/oder eine verfahrensgemäss erhältliche freie Verbindung in ein Salz oder eine verfahrensgemäss erhältliches Salz in die entsprechende freie Verbindung überführt.

23. Verwendung von Verbindungen der Formel XV 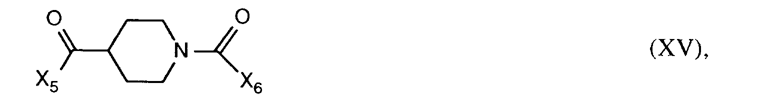 worin X₅ Halogen oder eine Gruppe der Formel X₇-O- darstellt, in der X₇ Niederalkansulfonyl, Niederalkoxycarbonyl, Niederalkanoyl oder eine Gruppe der Formel 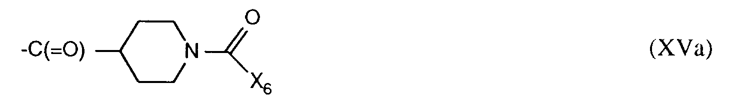 bedeutet, und X₆ für Niederalkoxy, Niederalkenyloxy oder gegebenenfalls durch Niederalkyl, Niederalkoxy, Halogen und/oder Nitro substituiertes Phenylniederalkoxy steht, in freier Form oder in Salzform zur Herstellung von Verbindungen der Formel 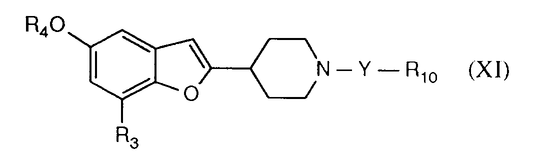 oder von Verbindungen der Formel 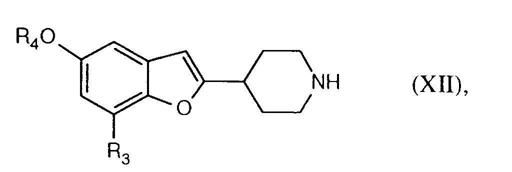 worin R₃ Halogen ist, R₄ Niederalkyl ist, Y für eine Gruppe -(C=O)- (Ie) und R₁₀ für eine Gruppe X₆ steht, und ihrer Salze.

24. Verfahren zur Herstellung von Verbindungen der Formel 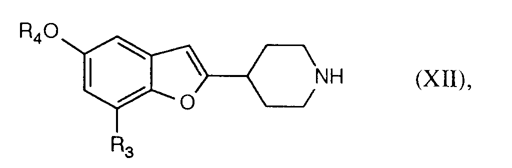 worin R₃ Halogen ist und R₄ Niederalkyl ist, und ihrer pharmazeutisch verwendbaren Salze, dadurch gekennzeichnet, dass man
g) eine Verbindung der Formel 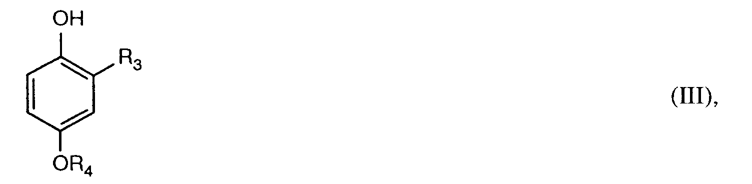 worin R₃ Halogen und R₄ Niederalkyl ist, oder ein Salz davon mit Paraformaldehyd oder Trioxan umsetzt und die erhaltene Verbindung der Formel 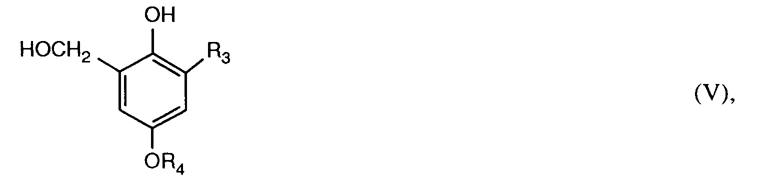 worin R₃ Halogen und R₄ Niederalkyl ist, in Gegenwart einer Halogenwasserstoffsäure der Formel HX₂, worin X₂ Halogen ist, mit einer Verbindung der Formel VIIc 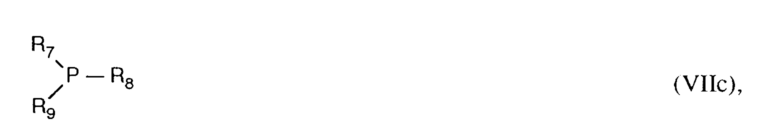 worin R₇, R₈ und R₉ jeweils unsubstituiertes substituiertes Phenyl darstellen, oder mit dem Säureadditionssalz einer Verbindung der Formel VIIc und einer Halogenwasserstoffsäure der Formel HX₂ umsetzt oder eine Verbindung der Formel 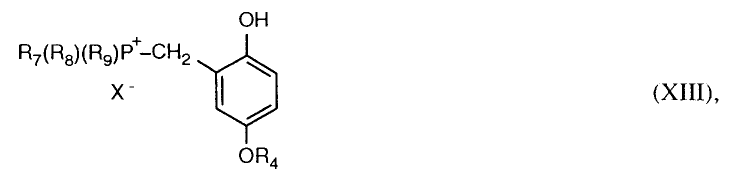 worin R₄ Niederalkyl ist und worin R₇, R₈ und R₉ jeweils unsubstituiertes Phenyl darstellen, durch Behandlung mit elementarem Halogen in einem Niederalkanol in m-Stellung zur Gruppe der Formel R₇(R₈)(R₉)P⁺CH₂- halogeniert, jeweils
h) die erhaltene Verbindung der Formel 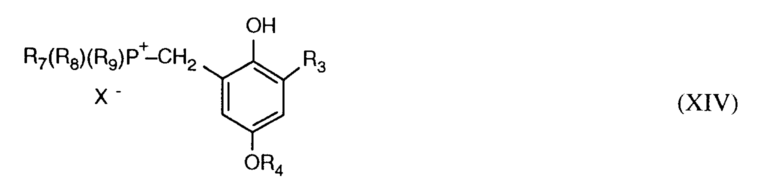 mit einer Verbindung der Formel 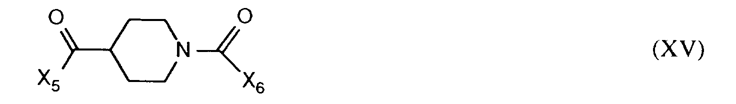 oder einem Salz davon umsetzt, worin X₅ Halogen oder eine Gruppe der Formel X₇-O-darstellt, in der X₇ Niederalkansulfonyl, Niederalkoxycarbonyl oder Niederalkoxycarbonyl ist oder eine Gruppe der Formel 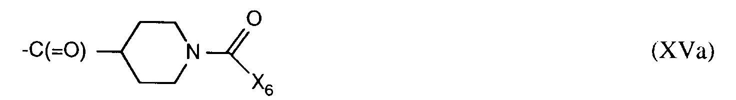 bedeutet, und X₆ für Niederalkoxy, Niederalkenyloxy oder gegebenenfalls durch Niederalkyl, Niederalkoxy, Halogen oder Nitro substituiertes Phenylniederalkoxy steht,
i) die erhaltene Verbindung der Formel 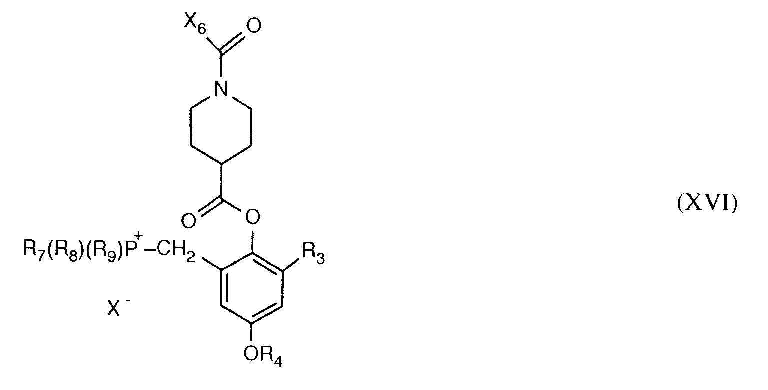 zur entsprechenden Verbindung der Formel 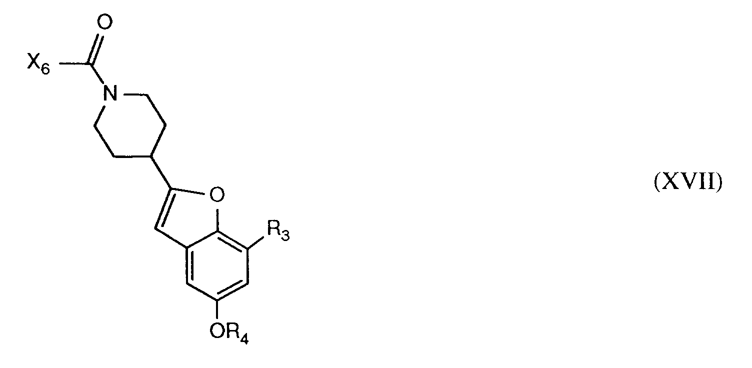 cyclisiert,
j) aus dieser die Gruppe der Formel -C(=O)-X₆ abspaltet und gewünschtenfalls eine erhaltene freie Verbindung in ein Säureadditionssalz oder ein erhaltenes Säureadditionssalz in die freie Verbindung oder in ein anderes Säureadditionssalz überführt.

## Claims

1. A compound of formula I 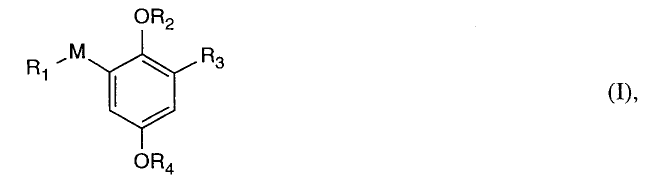 wherein R₁ is hydroxy, halogen, a group of the formula -P(=O)(R₅)R₆ (Ia) or a group of the formula -P⁺(R₇)(R₈)R₉ X⁻ (Ib), M is methylene, R₂ is hydrogen or a group of the formula 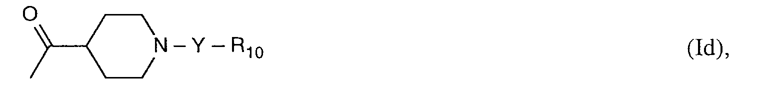 R₃ is hydrogen or halogen, R₄ is lower alkyl, each of R₅ and R₆, independently of the other, is lower alkyl, lower alkoxy or N,N-di-lower alkylamino, or is benzyl, benzyloxy, phenyl or phenoxy, each of which is unsubstituted or mono- or di-substituted at the phenyl ring, wherein the substituents of the phenyl ring may be selected in each case from the group consisting of lower alkyl, lower alkoxy, nitro and halogen, each of R₇, R₈ and R₉, independently of the others, is lower alkyl, unsubstituted or mono- or di-substituted phenyl, wherein the substituents of the phenyl ring may be selected in each case from the group consisting of lower alkyl, lower alkoxy, nitro and halogen, or is furyl, X⁻ is the anion of a hydrohalic acid, a lower alkanesulfonic acid, a halo-lower alkanesulfonic acid or of a benzenesulfonic acid that is unsubstituted or mono-substituted by lower alkyl or by halogen, and wherein either Y is a direct bond and R₁₀ is lower alkyl, allyl, cyano, lower alkanesulfonyl, halo-lower alkanesulfonyl, benzyl that is unsubstituted or mono- or di-substituted at the phenyl ring, wherein the substituents of the phenyl ring may be selected from the group consisting of lower alkyl, lower alkoxy, nitro and halogen, or is benzene-sulfonyl that is unsubstituted or mono-substituted by lower alkyl or by halogen, or Y is a group of the formula -(C=O)- (Ie) or a group of the formula -(C=S)- (If) and R₁₀ is halogen, lower alkyl, halo-lower alkyl, lower alkoxy, lower alkenyloxy, phenyl-lower alkenyloxy, halo-lower alkoxy or lower alkylthio, or is phenyl, phenoxy, phenylthio, benzyl, benzyloxy or benzylthio, each of which is unsubstituted or mono- or di-substituted at the phenyl ring, wherein the substituents of the phenyl ring may be selected in each case from the group consisting of lower alkyl, lower alkoxy, nitro and halogen, with the proviso that, in a compound I wherein R₁ is a group Ib wherein each of R₇, R₈ and R₉ is unsubstituted phenyl and X⁻ is the bromide ion, R₂ is hydrogen and R₄ is methyl, R₃ is other than hydrogen, and with the further proviso that, in a compound I wherein R₁ is hydroxy, R₂ is hydrogen and R₄ is methyl or ethyl, R₃ is other than hydrogen, in free form or in salt form.

2. A compound according to claim 1 of formula I, wherein R₁ is hydroxy, halogen, a group Ia or a group Ib, M is methylene, R₂ is hydrogen or a group Id, R₃ is hydrogen or halogen, and R₄ is lower alkyl, each of R₅ and R₆, independently of the other, is lower alkoxy, each of R₇, R₈ and R₉ is unsubstituted phenyl or furyl, X⁻ is the anion of a hydrohalic acid, Y is a group Ie and R₁₀ is lower alkoxy or lower alkenyloxy, in free form or in salt form.

3. A compound according to claim 1 of formula I, wherein R₁ is hydroxy or a group Ib, M is methylene, R₂ is hydrogen or a group Id, R₃ is halogen, R₄ is C₁-C₄alkyl, R₇, R₈ and R₉ are unsubstituted phenyl, X⁻ is the anion of a hydrohalic acid, Y is a group Ie and R₁₀ is C₁-C₄alkoxy, in free form or in salt form.

4. A compound according to claim 1 of formula I, wherein R₁ is a group Ib, M is methylene, R₂ is hydrogen or a group Id, R₃ is bromine, R₄ is methyl, each of R₇, R₈ and R₉ is unsubstituted phenyl, X⁻ is the chloride ion or the bromide ion, Y is a group Ie and R₁₀ is ethoxy, in free form or in salt form.

5. 3-Bromo-2-hydroxy-5-methoxy-benzyl alcohol according to claim 1.

6. [(3-Bromo-2-hydroxy-5-methoxy-phenyl)methyl]-triphenyl-phosphonium chloride according to claim 1.

7. [(3-Bromo-2-hydroxy-5-methoxy-phenyl)methyl]-triphenyl-phosphonium bromide according to claim 1.

8. [(3-Bromo-2-[(1-ethoxycarbonylpiperid-4-yl)carbonyloxy]-5-methoxy-phenyl)methyl]-triphenyl-phosphonium chloride according to claim 1.

9. 3-Bromo-2-hydroxy-5-methoxy-benzyl bromide according to claim 1.

10. [(3-Bromo-2-hydroxy-5-methoxy-phenyl)methyl]phosphonic acid dimethyl ester according to claim 1.

11. 3-Bromo-2-hydroxy-5-methoxy-benzoic acid methyl ester according to claim 1.

12. A process for the preparation of a compound of formula I according to claim 1, in free form or in salt form, wherein
a) for the preparation of a compound of formula I wherein R₁ is lower alkoxy, M is additionally carbonyl, R₂ is hydrogen, R₃ is halogen and R₄ is lower alkyl, or of a salt thereof, a compound of formula II 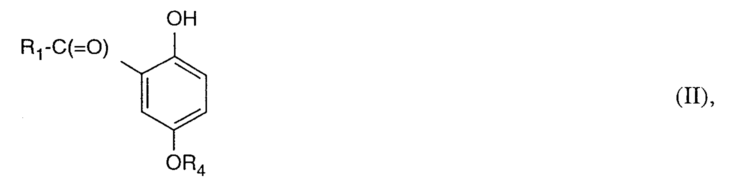 wherein R₁ is hydroxy or lower alkoxy and R₄ is lower alkyl, or a salt thereof, is halogenated in the m-position with respect to the R₁-C(=O)- group by treatment with elementary halogen in a lower alkanol, carboxy R₁-C(=O)-, where present, being esterified to lower alkoxycarbonyl, and/or
b) for the preparation of a compound of formula I wherein R₁ is hydroxy, M is methylene, R₂ is hydrogen, R₃ is hydrogen or halogen and R₄ is lower alkyl, with the proviso that R₃ is other than hydrogen when R₄ is methyl or ethyl, or of a salt thereof, a compound of formula III 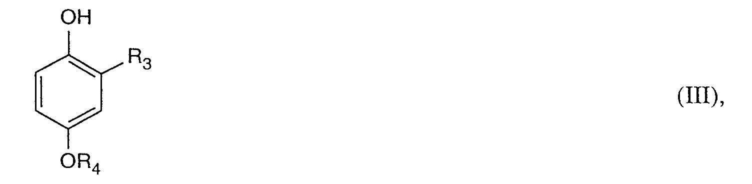 wherein R₃ is hydrogen or halogen and R₄ is lower alkyl, or a salt thereof, is reacted with paraformaldehyde or trioxane,
or in a compound of the formula 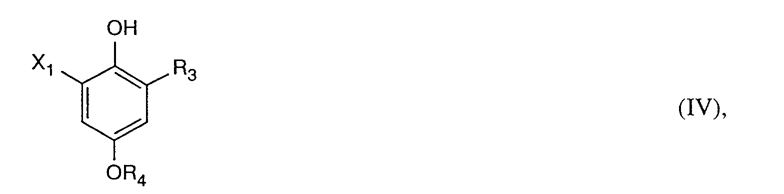 wherein X₁ is the formyl group, carboxy or lower alkoxycarbonyl, R₃ is halogen and R₄ is lower alkyl, or in a salt thereof, the group X₁ is reduced to hydroxymethyl, and/or
c) for the preparation of a compound of formula I wherein R₁ is halogen, M is methylene, R₂ is hydrogen and R₃ is halogen, or of a salt thereof, in a compound of the formula 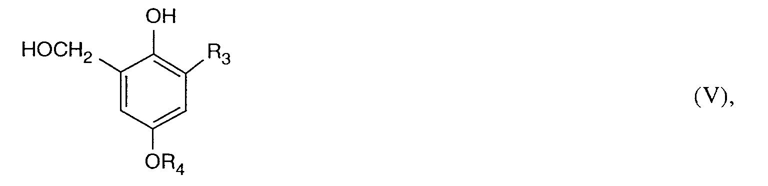 the hydroxymethyl group is converted into halomethyl, or a compound of the formula 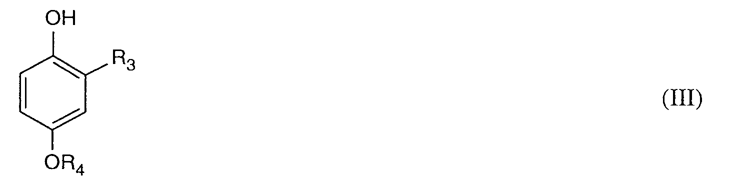 is reacted with paraformaldehyde or trioxane and a hydrohalic acid, and/or
d) for the preparation of a compound of formula I wherein R₁ is a group of the formula -P(=O)(R₅)R₆ (Ia) or a group of the formula -P⁺(R₇)(R₈)R₉ X⁻ (Ib), M is methylene, R₂ is hydrogen, R₃ is halogen and R₄ is lower alkyl and R₅, R₆, R₇, R₈, R₉ and X⁻ are as defined for formula I, or of a salt thereof, a compound of the formula 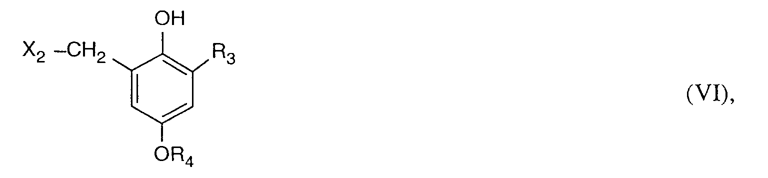 wherein X₂ is halogen, R₃ is halogen and R₄ is lower alkyl, is reacted with a compound of formula VIIa, VIIb or VIIc 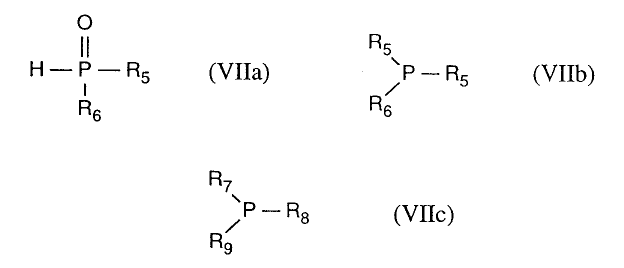 or with a salt thereof, wherein R₅, R₆, R₇, R₈ and R₉ are as defined for formula I, or a compound of the formula 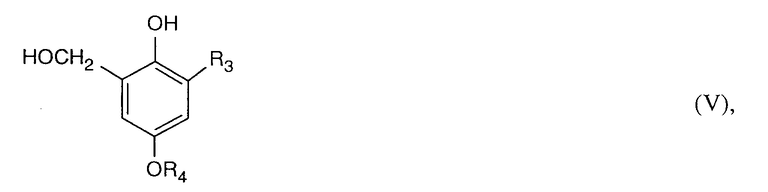 wherein R₃ is halogen and R₄ is lower alkyl, is reacted with a compound of formula VIIc 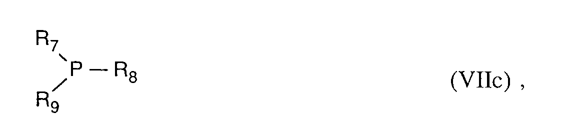 wherein R₇, R₈ and R₉ are as defined for formula I, and a hydrohalic acid of the formula HX, and/or
e) for the preparation of a compound of formula I wherein R₁ is a group of the formula -P(=O)(R₅)R₆ (Ia) or a group of the formula -P⁺(R₇)(R₈)R₉ X⁻ (Ib), M is methylene, R₂ is hydrogen, R₃ is halogen and R₄ is lower alkyl and R₅, R₆, R₇, R₈, R₉ and X⁻ are as defined for formula I, or of a salt thereof, a compound of the formula 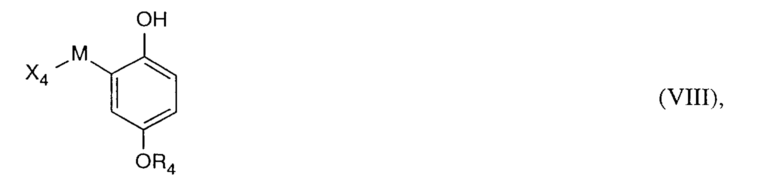 wherein X₄ is a group of the formula -P(=O)(R₅)R₆ (Ia) or a group of the formula -P⁺(R₇)(R₅)R₉ X⁻ (Ib), M is methylene and R₄ is lower alkyl, is halogenated in the m-position with respect to the X₄-M group, and/or
f) for the preparation of a compound of formula I wherein R₁ is a group of the formula -P⁺(R₇)(R₈)R₉ X⁻ (Ib), M is methylene, R₂ is a group of the formula 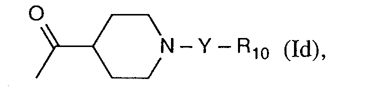 R₃ is halogen and R₄ is lower alkyl and R₇, R₈, R₉, R₁₀, Y and X⁻ are as defined for formula I, or of a salt thereof, a compound of the formula 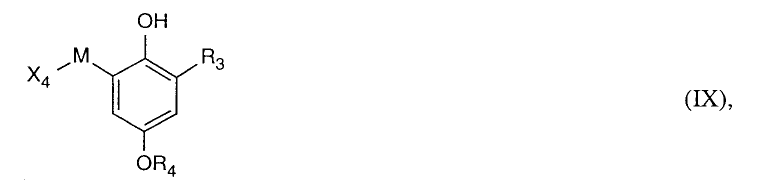 wherein X₄ is a group of the formula -P⁺(R₇)(R₈)R₉ X⁻ (Ib), M is methylene, R₃ is halogen and R₄ is lower alkyl, and R₇, R₈, R₉ and X⁻ are as defined for formula I, is reacted with a compound of the formula 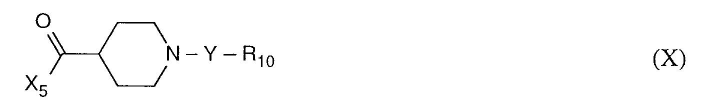 or with a salt thereof, wherein X₅ is halogen or a group of the formula X₇-O-, wherein X₇ denotes lower alkanesulfonyl, lower alkoxycarbonyl, lower alkanoyl or a group of the formula 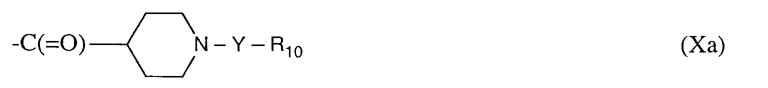 and R₁₀ and Y are as defined for formula I, and, if desired, a resulting compound is converted into a different compound of formula I, a mixture of isomers obtainable in accordance with the process is separated into its components and the preferred isomer in each case is isolated, and/or a free compound obtainable in accordance with the process is converted into a salt or a salt obtainable in accordance with the process is converted into the corresponding free compound.

13. The use of a compound of formula I 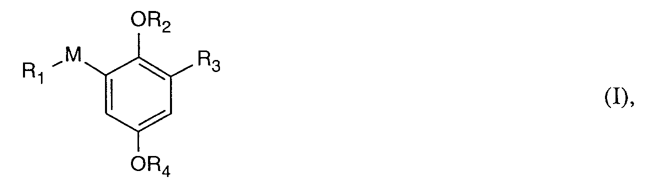 wherein R₁ is hydroxy, halogen, a group of the formula -P(=O)(R₅)R₆ (Ia) or a group of the formula -P⁺(R₇)(R₈)R₉ X⁻ (Ib), M is methylene, R₂ is hydrogen or a group of the formula 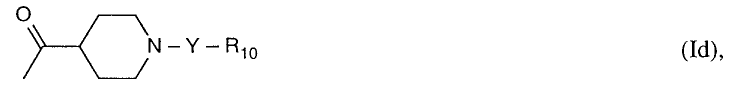 R₃ is hydrogen or halogen, R₄ is lower alkyl, each of R₅ and R₆, independently of the other, is lower alkyl, lower alkoxy or N,N-di-lower alkylamino, or is benzyl, benzyloxy, phenyl or phenoxy, each of which is unsubstituted or mono- or di-substituted at the phenyl ring, wherein the substituents of the phenyl ring may be selected in each case from the group consisting of lower alkyl, lower alkoxy, nitro and halogen, each of R₇, R₈ and R₉, independently of the others, is lower alkyl, unsubstituted or mono- or di-substituted phenyl, wherein the substituents of the phenyl ring may be selected in each case from the group consisting of lower alkyl, lower alkoxy, nitro and halogen, or is furyl, X⁻ is the anion of a hydrohalic acid, a lower alkanesulfonic acid, a halo-lower alkanesulfonic acid or of a benzenesulfonic acid that is unsubstituted or mono-substituted by lower alkyl or by halogen, and wherein either Y is a direct bond and R₁₀ is lower alkyl, allyl, cyano, lower alkanesulfonyl, halo-lower alkanesulfonyl, benzyl that is unsubstituted or mono- or di-substituted at the phenyl ring, wherein the substituents of the phenyl ring may be selected from the group consisting of lower alkyl, lower alkoxy, nitro and halogen, or is benzene-sulfonyl that is unsubstituted or mono-substituted by lower alkyl or by halogen, or Y is a group of the formula -(C=O)- (Ie) or a group of the formula -(C=S)- (If) and R₁₀ is halogen, lower alkyl, halo-lower alkyl, lower alkoxy, lower alkenyloxy, phenyl-lower alkenyloxy, halo-lower alkoxy or lower alkylthio, or is phenyl, phenoxy, phenylthio, benzyl, benzyloxy or benzylthio, each of which is unsubstituted or mono- or di-substituted at the phenyl ring, wherein the substituents of the phenyl ring may be selected in each case from the group consisting of lower alkyl, lower alkoxy, nitro and halogen, with the proviso that, in a compound I wherein R₁ is a group Ib wherein each of R₇, R₈ and R₉ is unsubstituted phenyl and X⁻ is the bromide ion, M is methylene, R₂ is hydrogen and R₄ is methyl, R₃ is other than hydrogen, and with the further proviso that in a compound I wherein R₁ is hydroxy, M is methylene, R₂ is hydrogen and R₄ is methyl or ethyl, R₃ is other than hydrogen, in free form or in salt form, for the preparation of a compound of the formula 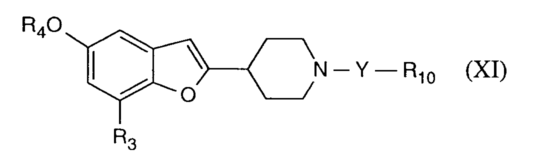 or of a compound of the formula 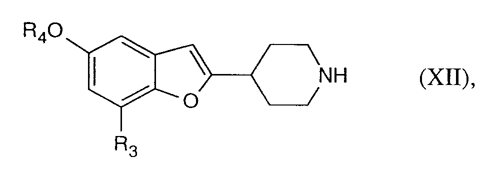 wherein R₃ is halogen, R₄ is lower alkyl and R₁₀ and Y are as defined, or of a salt of each of those compounds.

14. A compound of formula XV 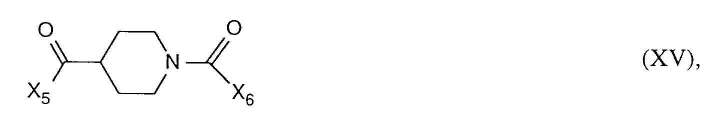 wherein X₅ is halogen or a group of the formula X₇-O-, wherein X₇ denotes lower alkane-sulfonyl, lower alkoxycarbonyl, lower alkanoyl or a group of the formula 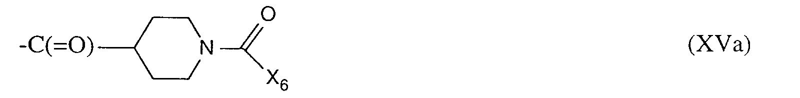 and X₆ stands for lower alkoxy, lower alkenyloxy or phenyl-lower alkoxy that is unsubstituted or substituted by lower alkyl, lower alkoxy, halogen or nitro, with the proviso that X₆ is other than unsubstituted benzyl when X₅ is chlorine, or a salt thereof.

15. A compound according to claim 14 of formula XV, wherein X₅ represents halogen and X₆ stands for lower alkoxy, or a salt thereof.

16. 4-Chlorocarbonyl-1-ethoxycarbonyl-piperidine according to claim 14.

17. 4-Chlorocarbonyl-1-allyloxycarbonyl-piperidine according to claim 14.

18. 1-Ethoxycarbonyl-4-methanesulfonyloxycarbonyl-piperidine according to claim 14.

19. 1-Ethoxycarbonyl-4-isobutyloxycarbonyloxycarbonyl-piperidine according to claim 14.

20. 1-Ethoxycarbonyl-4-pivaloyloxycarbonyl-piperidine according to claim 14.

21. 1,1'-Bis(ethoxycarbonyl)piperidine-4-carboxylic anhydride according to claim 14.

22. A process for the preparation of a compound of formula XV 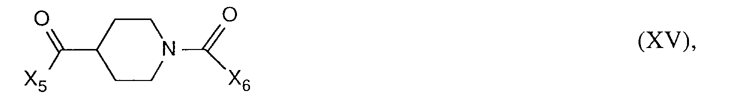 wherein X₅ is halogen or a group of the formula X₇-O-, wherein X₇ denotes lower alkane-sulfonyl, lower alkoxycarbonyl, lower alkanoyl or a group of the formula 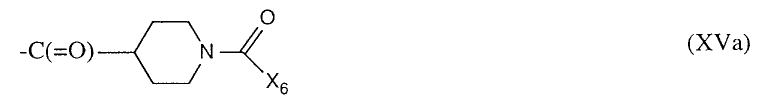 and X₆ stands for lower alkoxy, lower alkenyloxy or phenyl-lower alkoxy that is unsubstituted or substituted by lower alkyl, lower alkoxy, halogen and/or nitro, with the proviso that X₆ is other than unsubstituted benzyl when X₅ is chlorine, or of a salt thereof, wherein a compound of formula XVIII 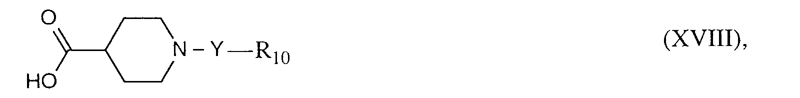 wherein Y and R₁₀ are as defined hereinbefore, or a salt thereof, is reacted with an agent that introduces the group X₅ and, if desired, a resulting compound is converted into a different compound of formula XV and/or a free compound obtainable in accordance with the process is converted into a salt or a salt obtainable according to the process is converted into the corresponding free compound.

23. The use of a compound of formula XV 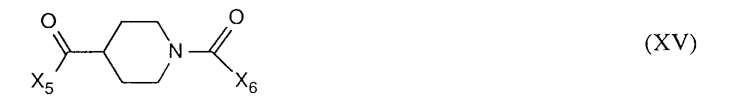 wherein X₅ is halogen or a group of the formula X₇-O-, wherein X₇ denotes lower alkane-sulfonyl, lower alkoxycarbonyl, lower alkanoyl or a group of the formula 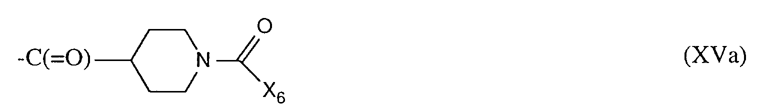 and X₆ stands for lower alkoxy, lower alkenyloxy or phenyl-lower alkoxy that is unsubstituted or substituted by lower alkyl, lower alkoxy, halogen and/or nitro, in free form or in salt form, for the preparation of a compound of the formula 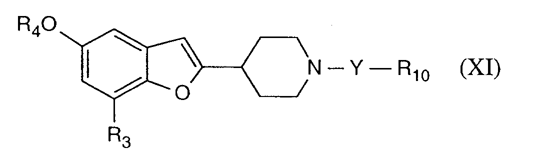 or of a compound of the formula 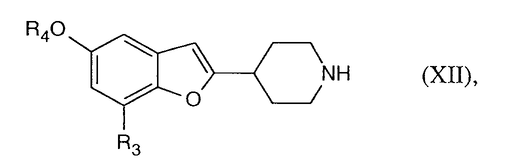 wherein R₃ denotes halogen, R₄ is lower alkyl, Y stands for a group -(C=O)- (Ie) and R₁₀ denotes a group X₆, or of a salt of each of those compounds.

24. A process for the preparation of a compound of the formula 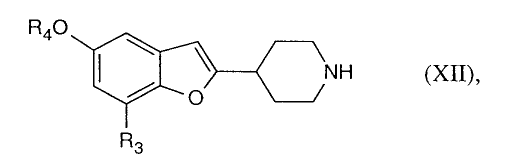 wherein R₃ is halogen and R₄ is lower alkyl, or of a pharmaceutically acceptable salt thereof, wherein
g) a compound of the formula 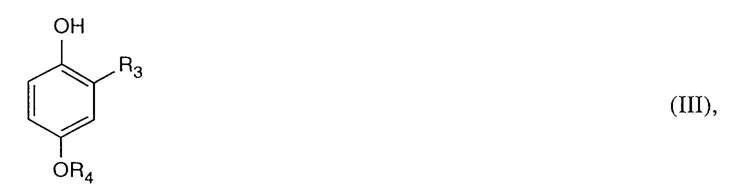 wherein R₃ is halogen and R₄ is lower alkyl, or a salt thereof, is reacted with paraform-aldehyde or trioxane and the resulting compound of the formula 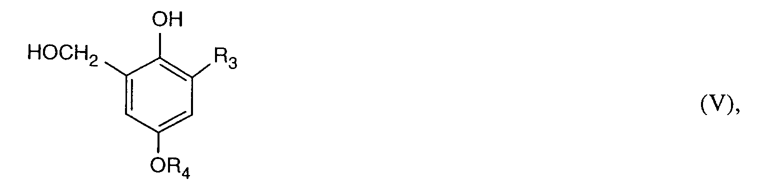 wherein R₃ is halogen and R₄ is lower alkyl, is reacted in the presence of a hydrohalic acid of the formula HX₂, wherein X₂ is halogen, with a compound of formula VIIc 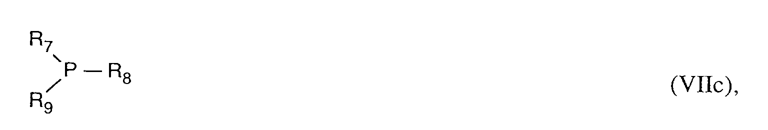 wherein each of R₇, R₈ and R₉ is unsubstituted or substituted phenyl, or with the acid addition salt of a compound of formula VIIc and a hydrohalic acid of the formula HX₂, or a compound of the formula 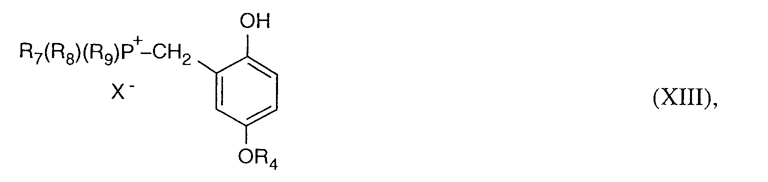 wherein R₄ is lower alkyl and wherein each of R₇, R₈ and R₉ is unsubstituted phenyl, is halogenated in the m-position with respect to the group of the formula R₇(R₈)(R₉)P⁺CH₂- by treatment with elementary halogen in a lower alkanol, and in each case
h) the resulting compound of the formula 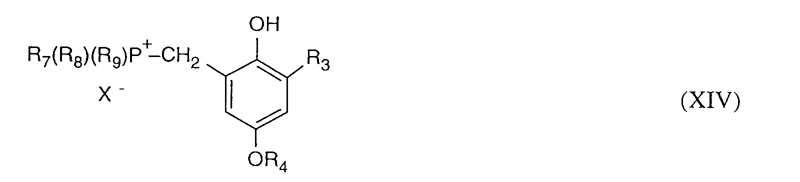 is reacted with a compound of the formula 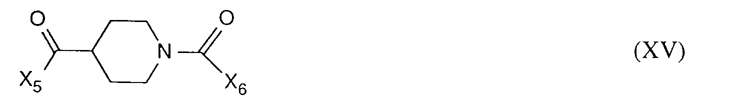 or with a salt thereof, wherein X₅ is halogen or a group of the formula X₇-O-, wherein X₇ is lower alkanesulfonyl, lower alkoxycarbonyl or lower alkoxycarbonyl or denotes a group of the formula 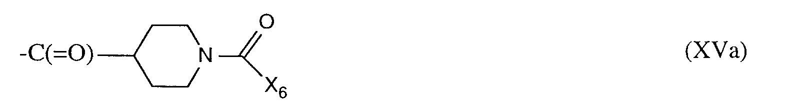 and X₆ stands for lower alkoxy, lower alkenyloxy or phenyl-lower alkoxy that is unsubstituted or substituted by lower alkyl, lower alkoxy, halogen and/or nitro,
i) the resulting compound of the formula 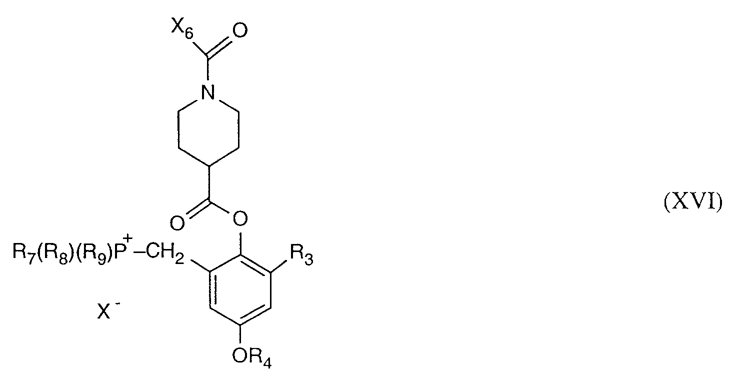 is cyclised to form the corresponding compound of the formula 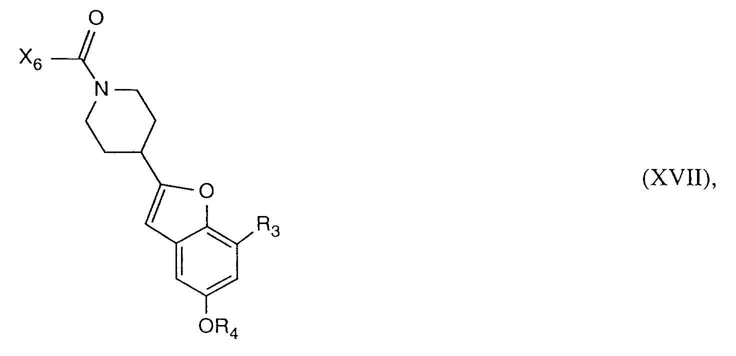 and
j) the group of the formula -C(=O)-X₆ is removed from that compound, and, if desired, a resulting free compound is converted into an acid addition salt or a resulting acid addition salt is converted into the free compound or into a different acid addition salt.

## Revendications

1. Un composé de formule I 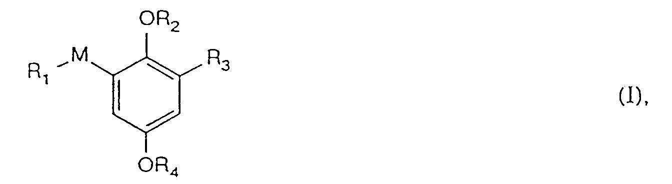 où R₁ est l'hydroxy, un halogène, un groupe de formule -P(=O)(R₅)R₆ (Ia) ou un groupe de formule -P⁺(R₇)(R₈)R₉X- (Ib), M est le méthylène, R₂ est l'hydrogène ou un groupe de formule 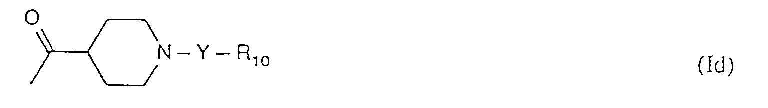 R₃ est l'hydrogène ou un halogène, R₄ est un alkyle inférieur, R₅ et R₆ représentent, indépendamment l'un de l'autre, un alkyle inférieur, un alcoxy inférieur, un N,N-dialkyle inférieur amino ou le benzyle, le benzyloxy, le phényle ou le phényloxy, non substitués ou mono- ou disubstitués sur le noyau phénylique, les substituants du noyau phénylique pouvant être choisis dans le groupe constitué par les alkyles inférieurs, les alcoxy inférieurs, le nitro et les halogènes, R₇, R₈ et R₉ représentent, indépendamment les uns des autres, un alkyle inférieur, le phényle non substitué ou mono- ou disubstitué, les substituants du noyau phénylique pouvant être choisis dans le groupe constitué par les alkyles inférieurs, les alcoxy inférieurs, le nitro et les halogènes, ou représentent le furyle, X⁻ est l'anion d'un hydracide halogéné, d'un acide alcane inférieur sulfonique, d'un acide halogénoalcane inférieur sulfonique ou d'un acide benzènesulfonique non substitué ou monosubstitué par un alkyle inférieur ou un halogène et où ou Y représente une liaison directe et R₁₀ est un alkyle inférieur, l'allyle, le cyano, un alcane inférieur sulfonyle, un halogénoalcane inférieur sulfonyle, le benzyle non substitué ou mono- ou disubstitué sur le noyau phénylique, les substituants du noyau phénylique pouvant être choisis dans le groupe constitué par les alkyles inférieurs, les alcoxy inférieurs, le nitro et les halogènes, ou est le benzènesulfonyle non substitué ou monosubstitué par un alkyle inférieur ou un halogène ou où Y est un groupe de formule -(C=O)- (Ie) ou un groupe de formule -(C=S)- (If) et R₁₀ est un halogène, un alkyle inférieur, un halogénoalkyle inférieur, un alcoxy inférieur, un alcényloxy inférieur, un phénylalcényloxy inférieur, un halogénoalcoxy inférieur, un alkyle inférieur thio ou le phényl, le phényloxy, le phénylthio, le benzyle, le benzyloxy ou le benzylthio, non substitués ou mono- ou disubstitués sur le noyau phénylique, les substituants du noyau phénylique pouvant être choisis dans le groupe constitué par les alkyles inférieurs, les alcoxy inférieurs, le nitro et les halogènes, sous réserve que dans un composé I où R₁ est un groupe Ib dans lequel R₇, R₈ et R₉ représentent le phényle non substitué et X⁻ l'ion bromure, R₂ est l'hydrogène et R₄ le méthyle, R₃ est différent de l'hydrogène et sous réserve encore que dans un composé I où R₁ est l'hydroxy, R₂ l'hydrogène et R₄ le méthyle ou l'éthyle, R₃ est différent de l'hydrogène, sous forme libre ou sous forme de sel.

2. Un composé selon la revendication 1 de formule I où R₁ est l'hydroxy, un halogène, un groupe Ia ou un groupe Ib, M est le méthylène, R₂ est l'hydrogène ou un groupe Id, R₃ est l'hydrogène ou un halogène et R₄ est un alkyle inférieur, R₅ et R₆ représentent, indépendamment l'un de l'autre, un alcoxy inférieur, R₇, R₈ et R₉ représentent le phényle non substitué ou le furyle, X⁻ est l'anion d'un hydracide halogéné, Y est un groupe Ie et R₁₀ est un alcoxy inférieur ou un alcényloxy inférieur, sous forme libre ou sous forme de sel.

3. Un composé selon la revendication 1 de formule I où R₄ est l'hydroxy ou un groupe Ib, M est le méthylène, R₂ est l'hydrogène ou un groupe Id, R₃ est un halogène, R₄ est un alkyle en C₁-C₄, R₇, R₈ et R₉ représentent le phényle non substitué, X⁻ est l'anion d'un hydracide halogéné, Y est un groupe Ie et R₁₀ est un alcoxy en C₁-C₄, sous forme libre ou sous forme de sel.

4. Un composé selon la revendication 1 de formule I où R₁ est un groupe Ib, M est le méthylène, R₂ est l'hydrogène ou un groupe Id, R₃ est le brome, R₄ est le méthyle, R₇, R₈ et R₉ représentent le phényle non substitué, X₋ est l'ion chlorure ou l'ion bromure, Y est un groupe Ie et R₁₀ est l'éthoxy, sous forme libre ou sous forme de sel.

5. L'alcool 3-bromo-2-hydroxy-5-méthoxy-benzylique selon la revendication 1.

6. Le chlorure de [(3-bromo-2-hydroxy-5-méthoxy-phényl)méthyl]-triphényl-phosphonium selon la revendication 1.

7. Le bromure de [(3-bromo-2-hydroxy-5-méthoxy-phényl)méthyl]-triphényl-phosphonium selon la revendication 1.

8. Le bromure de [(3-bromo-2-[(1-éthoxycarbonylpipéridi-4-yl)carbonyloxy]-5-méthoxy-phényl)méthyl]-triphényl-phosphonium selon la revendication 1.

9. Le bromure de 3-bromo-2-hydroxy-5-méthoxy-benzyle selon la revendication 1.

10. L'ester diméthylique de l'acide [(3-bromo-2-hydroxy-5-méthoxy-phényl)méthyl]phosphonium selon la revendication 1.

11. L'ester méthylique de l'acide 3-bromo-2-hydroxy-5-méthoxy-benzoïque selon la revendication 1.

12. Procédé pour la préparation des composés de formule I selon la revendication 1, sous forme libre ou sous forme de sel, caractérisé
a) en ce que pour la préparation d'un composé de formule I où R₁ est un alcoxy inférieur, M représente en plus le carbonyle, R₂ est l'hydrogène, R₃ est un halogène et R₄ un alkyle inférieur ou de l'un de ses sels, l'on halogène un composé de formule II 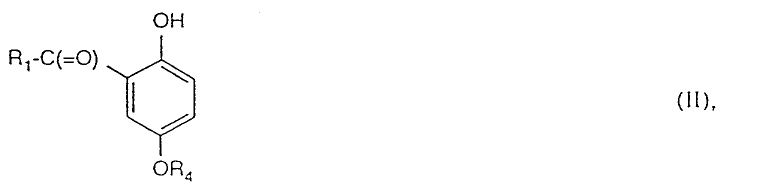 où R₁ représente l'hydroxy ou un alcoxy inférieur et R₄ un alkyle inférieur, ou l'un de ses sels, par traitement avec un halogène élémentaire dans un alcanol inférieur, en position méta par rapport au groupe R₁-C(=O), le carboxy R₁-C(=O)-, s'il est présent, étant estérifié en alcoxy inférieur carbonyle et/ou
b) en ce que pour la préparation d'un composé de formule I où R₁ est l'hydroxy, M le méthylène, R₂ l'hydrogène, R₃ l'hydrogène ou un halogène et R₄ un alkyle inférieur, sous réserve que lorsque R₄ représente le méthyle ou l'éthyle, R₃ est différent de l'hydrogène ou de l'un de ses sels, l'on fait réagir un composé de formule 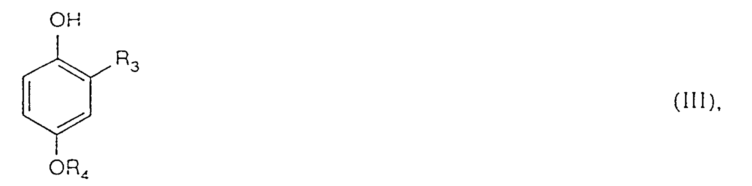 où R₃ est l'hydrogène ou un halogène et R₄ un alkyle inférieur ou l'un de ses sels, avec le paraformaldéhyde ou le trioxanne ou en ce que l'on réduit dans un composé de formule 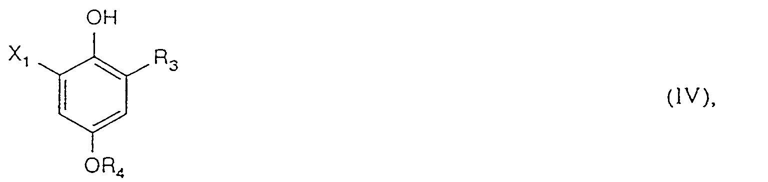 où X₁ représente le groupe formyle, le carboxy ou un alcoxy inférieur carbonyle, R₃ est un halogène et R₄ un alkyle inférieur, ou dans l'un de ses sels, le groupe X₁ en hydroxyméthyle et/ou
c) en ce que pour la préparation d'un composé de formule I où R₁ est un halogène, M le méthylène, R₂ l'hydrogène et R₃ un halogène ou de l'un de ses sels, l'on transforme dans un composé de formule 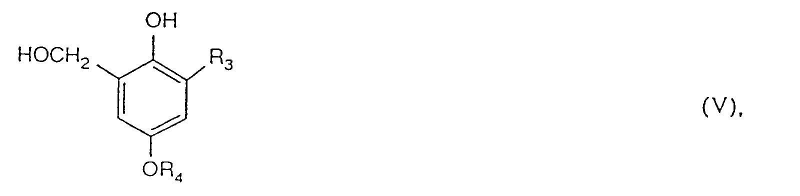 le groupe hydroxyméthyle en halogénométhyle ou l'on fait réagir un composé de formule 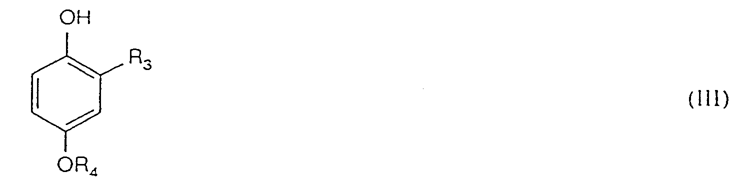 avec le paraformaldéhyde ou le trioxanne et un hydracide halogéné et/ou
d) en ce que pour la préparation d'un composé de formule I où R₁ est un groupe de formule -P(=O)(R₅)R₆ (Ia) ou un groupe de formule -P⁺(R₇)(R₈)R₉X⁻ (Ib), M est le méthylène, R₂ est l'hydrogène, R₃ un halogène, R₄ un alkyle inférieur et R₅, R₆, R₇, R₈, R₉ et X⁻ ont les significations indiquées pour la formule I ou de l'un de ses sels, l'on fait réagir un composé de formule 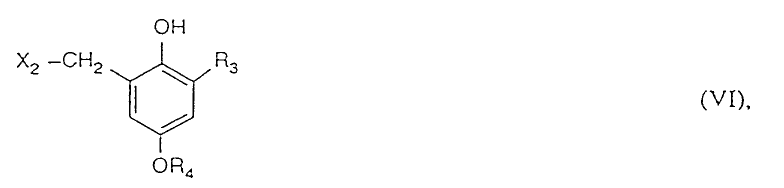 où X₂ est un halogène, R₃ est un halogène et R₄ un alkyle inférieur, avec un composé de formules VIIa, VIIb ou VIIc 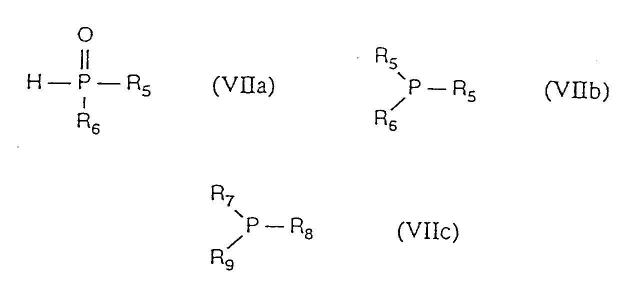 ou l'un de ses sels, où R₅, R₆, R₇, R₈ et R₉ ont les significations indiquées pour la formule I ou l'on fait réagir un composé de formule 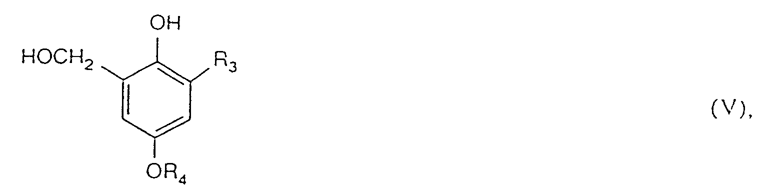 où R₃ est un halogène et R₄ un alkyle inférieur, avec un composé de formule VIIc 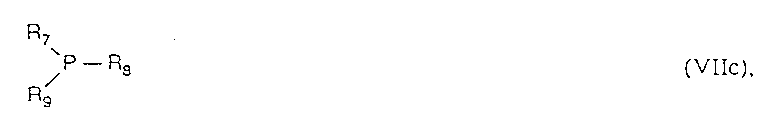 où R₇, R₈ et R₉ ont les significations indiquées pour la formule I, et un hydracide halogéné de formule HX et/ou
e) en ce que la préparation d'un composé de formule I où R₁ est un groupe de formule -P(=O)(R₅)R₆ (Ia) ou un groupe de formule -P⁺(R₇)(R₈)R₉X⁻ (Ib), M est le méthylène, R₂ l'hydrogène, R₃ un halogène, R₄ un alkyle inférieur et R₅, R₆, R₇, R₈, R₉ et X⁻ ont les significations indiquées pour la formule I ou de l'un de ses sels, l'on halogène un composé de formule 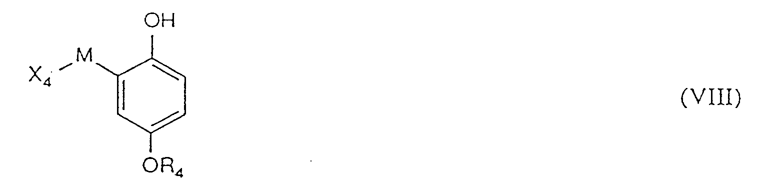 où X₄ est un groupe de formule -P(=O)(R₅)R₆ (Ia) ou un groupe de formule -P⁺(R₇)(R₈)R₉X⁻ (Ib), M le méthylène et R₄ un alkyle inférieur, en position méta par rapport au groupe X₄-M et/ou
f) en ce que la préparation d'un composé de formule I où R₁ est un groupe de formule -P⁺(R₇)(R₈)R₉X⁻ (Ib), M le méthylène, R₂ un groupe de formule 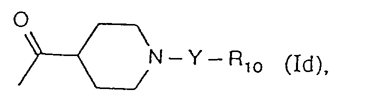 R₃ un halogène, R₄ un alkyle inférieur et R₇, R₈, R₉, R₁₀, Y et X⁻ ont les significations indiquées pour la formule I ou de l'un de ses sels, l'on fait réagir un composé de formule 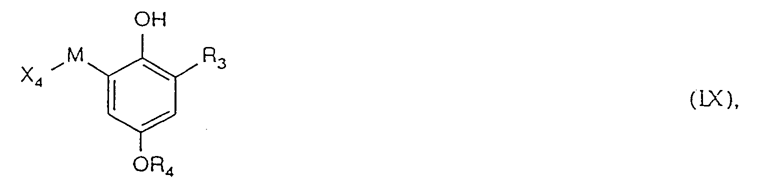 où X₄ est un groupe de formule -P⁺(R₇)(R₈)R₉X⁻ (Ib), M le méthylène, R₃ un halogène, R₄ un alkyle inférieur, et R₇, R₈, R₉ et X⁻ ont les significations indiquées pour la formule I, avec un composé de formule 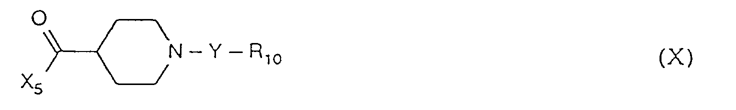 ou l'un de ses sels, où X₅ représente un halogène ou un groupe de formule X₇-O- dans laquelle X₇ est un alcane inférieur sulfonyle, un alcoxy inférieur carbonyle, un alcanoyle inférieur ou un groupe de formule 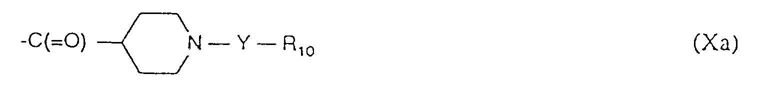 R₁₀ et Y ont les significations indiquées pour la formule I et éventuellement l'on transforme le composé obtenu ou un autre composé de formule I, l'on sépare le mélange d'isomères obtenu conformément au procédé de l'invention en ses composants, l'on sépare l'isomère préféré et/ou l'on transforme le composé libre obtenu conformément au procédé de l'invention en un sel ou le sel obtenu conformément au procédé de l'invention en un composé libre correspondant.

13. Utilisation des composés de formule I 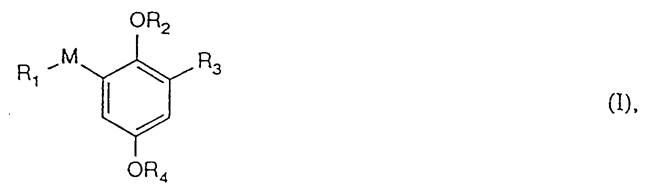 où R₁ est l'hydroxy, un halogène, un groupe de formule -P(=O)(R₅)R₆ (Ia) ou un groupe de formule -P⁺(R₇)(R₈)R₉X- (Ib), M est le méthylène, R₂ est l'hydrogène ou un groupe de formule 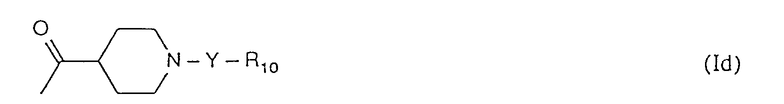 R₃ est l'hydrogène ou un halogène, R₄ est un alkyle inférieur, R₅ et R₆ représentent, indépendamment l'un de l'autre, un alkyle inférieur, un alcoxy inférieur, un N,N-dialkyle inférieur amino ou le benzyle, le benzyloxy, le phényle ou le phényloxy, non substitués ou mono- ou disubstitués sur le noyau phénylique, les substituants du noyau phénylique pouvant être choisis dans le groupe constitué par les alkyles inférieurs, les alcoxy inférieurs, le nitro et les halogènes, R₇, R₈ et R₉ représentent, indépendamment les uns des autres, un alkyle inférieur, le phényle non substitué ou mono- ou disubstitué, les substituants du noyau phénylique pouvant être choisis dans le groupe constitué par les alkyles inférieurs, les alcoxy inférieurs, le nitro et les halogènes, ou représentent le furyle, X⁻ est l'anion d'un hydracide halogéné, d'un acide alcane inférieur sulfonique, d'un acide halogénoalcane inférieur sulfonique ou d'un acide benzènesulfonique non substitué ou monosubstitué par un alkyle inférieur ou un halogène et où ou Y représente une liaison directe et R₁₀ est un alkyle inférieur, l'allyle, le cyano, un alcane inférieur sulfonyle, un halogénoalcane inférieur sulfonyle, le benzyle non substitué ou mono- ou disubstitué sur le noyau phénylique, les substituants du noyau phénylique pouvant être choisis dans le groupe constitué par les alkyles inférieurs, les alcoxy inférieurs, le nitro et les halogènes, ou est le benzènesulfonyle non substitué ou monosubstitué par un alkyle inférieur ou un halogène ou où Y est un groupe de formule -(C=O)- (Ie) ou un groupe de formule -(C=S)- (If) et R₁₀ est un halogène, un alkyle inférieur, un halogénoalkyle inférieur, un alcoxy inférieur, un alcényloxy inférieur, un phénylalcényloxy inférieur, un halogénoalcoxy inférieur, un alkyle inférieur thio ou le phényl, le phényloxy, le phénylthio, le benzyle, le benzyloxy ou le benzylthio, non substitués ou mono- ou disubstitués sur le noyau phénylique, les substituants du noyau phénylique pouvant être choisis dans le groupe constitué par les alkyles inférieurs, les alcoxy inférieurs, le nitro et les halogènes, sous réserve que dans un composé I où R₁ est un groupe Ib dans lequel R₇, R₈ et R₉ représentent le phényle non substitué et X⁻ l'ion bromure, M est le méthylène, R₂ est l'hydrogène et R₄ le méthyle, R₃ est différent de l'hydrogène et sous réserve encore que dans un composé I où R₁ est l'hydroxy, M est le méthylène, R₂ l'hydrogène et R₄ le méthyle ou l'éthyle, R₃ est différent de l'hydrogène, sous forme libre ou sous forme de sel, pour la préparation des composés de formule 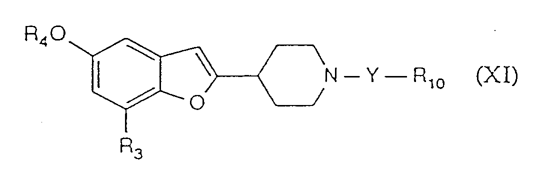 ou des composés de formule 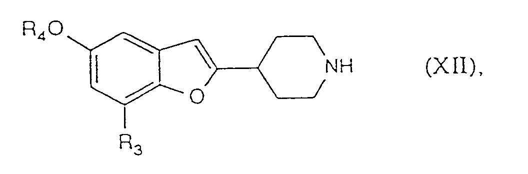 où R₃ est un halogène, R₄ est un alkyle inférieur et R₁₀ et Y ont les significations indiquées et de leurs sels.

14. Composés de formule XV 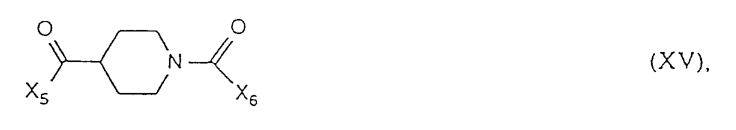 où X₅ représente un halogène ou un groupe de formule X₇-O-dans laquelle X₇ représente un alcane inférieur sulfonyle, un alcoxy inférieur carbonyle, un alcanoyle inférieur ou un groupe de formule 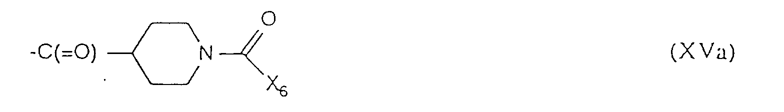 et X₆ un alcanoyle inférieur, un alcényloxy inférieur ou un phénylalcoxy inférieur éventuellement substitué par un alkyle inférieur, un alcoxy inférieur, un halogène ou le nitro, sous réserve que lorsque X₅ est le chlore, X₆ est différent du benzyle non substitué et leurs sels.

15. Composés selon la revendication 14 de formule XV où X₅ représente un halogène et X₆ représente un alcoxy inférieur et leurs sels.

16. La 4-chlorocarbonyl-1-éthoxycarbonyl-pipéridine selon la revendication 14.

17. La 4-chlorocarbonyl-1-allyloxycarbonyl-pipéridine selon la revendication 14.

18. La 1-éthoxycarbonyl-4-méthanesulfonyloxycarbonyl-pipéridine selon la revendication 14.

19. La1-éthoxycarbonyl-4-isobutyloxycarbonyloxycarbonyl-pipéridine selon la revendication 14.

20. La 1-éthoxycarbonyl-4-pivaloyloxycarbonyl-pipéridine selon la revendication 14.

21. L'anhydride de l'acide 1,1'-bis(éthoxycarbonyl)pipéridine-4-carboxylique selon la revendication 14.

22. Procédé pour la préparation des composés de formule XV 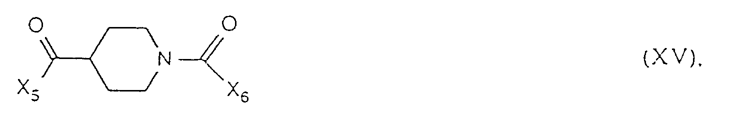 où X₅ représente un halogène ou un groupe de formule X₇-O-dans laquelle X₇ représente un alcane inférieur sulfonyle, un alcoxy inférieur carbonyle, un alcanoyle inférieur ou un groupe de formule 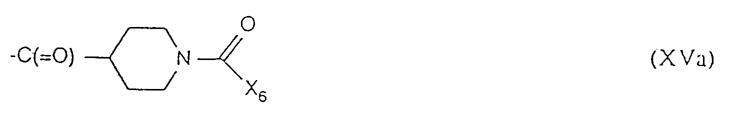 et X₆ représente un alcoxy inférieur, un alcényloxy inférieur ou un phénylalcoxy inférieur éventuellement substitué par un alkyle inférieur, un alcoxy inférieur, un halogène et/ou le nitro, sous réserve que lorsque X₅ est le chlore, X₆ est différent du benzyle non substitué et de leurs sels, caractérisé en ce que l'on fait réagir un composé de formule XVIII 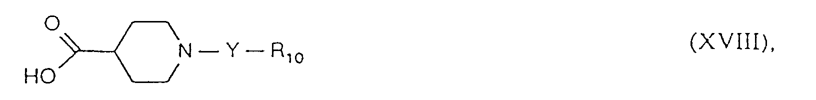 où Y et R₁₀ ont les significations indiquées ou l'un de ses sels avec un agent introduisant le reste X₅ et, si désiré, en ce que l'on transforme le composé obtenu et un autre composé de formule XV et/ou en ce que l'on transforme le composé libre obtenu conformément au procédé de l'invention en un sel ou le sel obtenu conformément au procédé de l'invention en un composé libre correspondant.

23. Utilisation des composés de formule XV 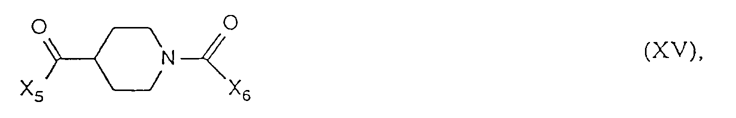 où X₅ représente un halogène ou un groupe de formule X₇-O- dans laquelle X₇ représente un alcane inférieur sulfonyle, un alcoxy inférieur carbonyle, un alcanoyle inférieur ou un groupe de formule 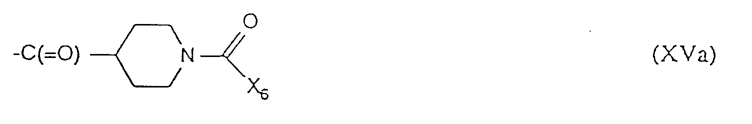 et X₆ représente un alcoxy inférieur, un alcényloxy inférieur ou un phénylalcoxy inférieur éventuellement substitué par un alkyle inférieur, un alcoxy inférieur, un halogène et/ou le nitro, sous forme libre ou sous forme de sel, pour la préparation des composés de formule 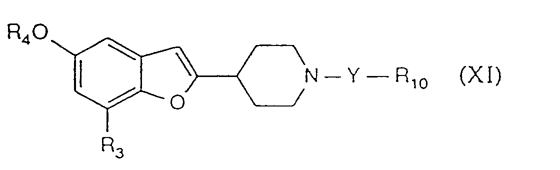 ou des composés de formule 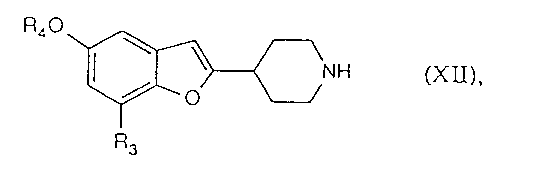 où R₃ est un halogène, R₄ est un alkyle inférieur, Y représente un groupe -(C=O)- (Ie) et R₁₀ représente un groupe X₆ et de leurs sels.

24. Procédé pour la préparation des composés de formule 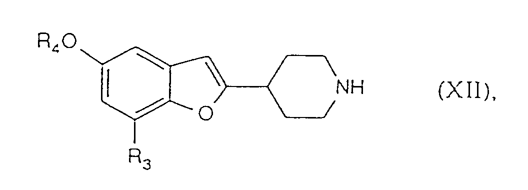 où R₃ est un halogène et R₄ est un alkyle inférieur et de leurs sels pharmaceutiquement utilisables, caractérisé
g) en ce que l'on fait réagir un composé de formule 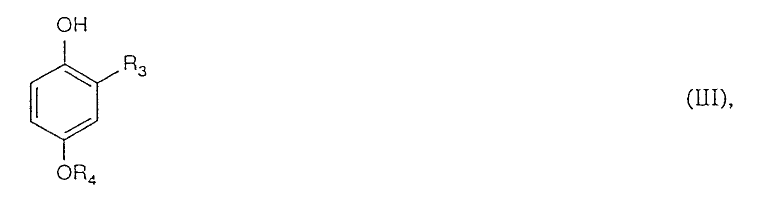 où R₃ représente un halogène et R₄ un alkyle inférieur ou l'un de ses sels avec le paraformaldéhyde ou le trioxanne et en ce que l'on fait réagir le composé obtenu de formule 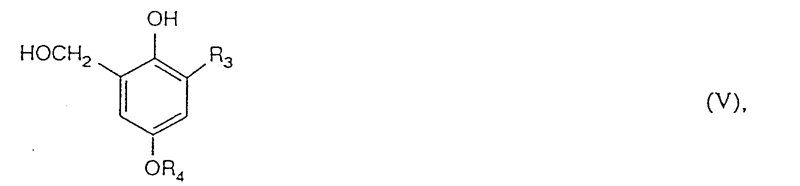 où R₃ est un halogène et R₄ un alkyle inférieur, en présence d'un hydracide halogéné de formule HX₂, X₂ étant un halogène, avec un composé de formule VIIc 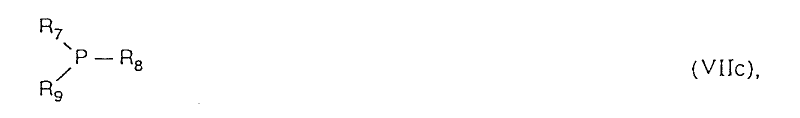 où R₇, R₈ et R₉ représentent le phényle non substitué ou avec le sel d'addition d'acide d'un composé de formule VIIc et d'un hydracide halogéné de formule HX₂ où X₂ est un halogène ou en ce que l'on halogène un composé de formule 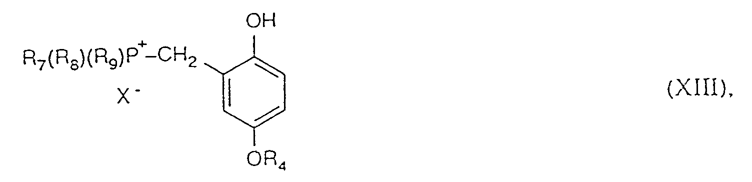 où R₄ est un alkyle inférieur et où R₇, R₈ et R₉ représentent le phényl non substitué, par traitement avec un halogène élémentaire dans un alcanol inférieur, en position méta par rapport au groupe de formule R₇(R₈)(R₉)P⁺CH₂-,
h) en ce que l'on fait réagir le composé obtenu de formule 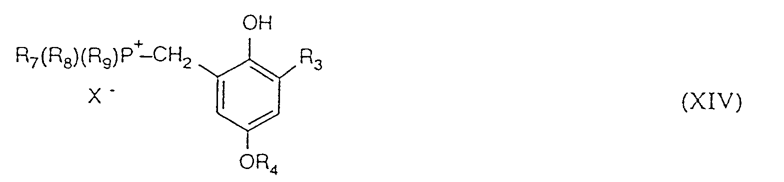 avec un composé de formule 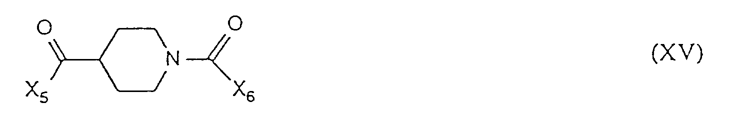 ou l'un de ses sels, où X₅ représente un halogène ou un groupe de formule X₇-O- dans laquelle X₇ est un alcane inférieur sulfonyle, un alcoxy inférieur carbonyle ou un alcoxy inférieur carbonyle ou représente un groupe de formule 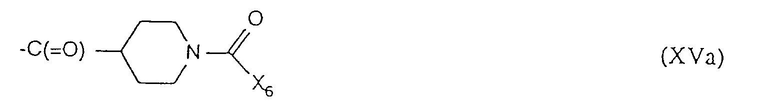 et X₆ représente un alcoxy inférieur, un alcényloxy inférieur ou un phénylalcoxy inférieur éventuellement substitué par un alkyle inférieur, un alcoxy inférieur, un halogène ou le nitro,
i) en ce que l'on cyclise le composé obtenu de formule 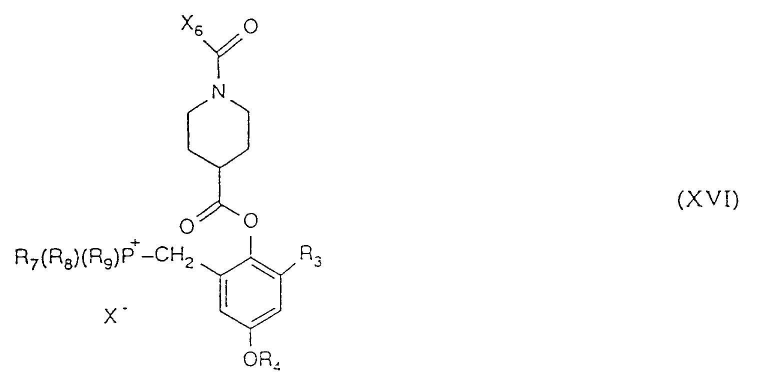 pour former le composé correspondant de formule 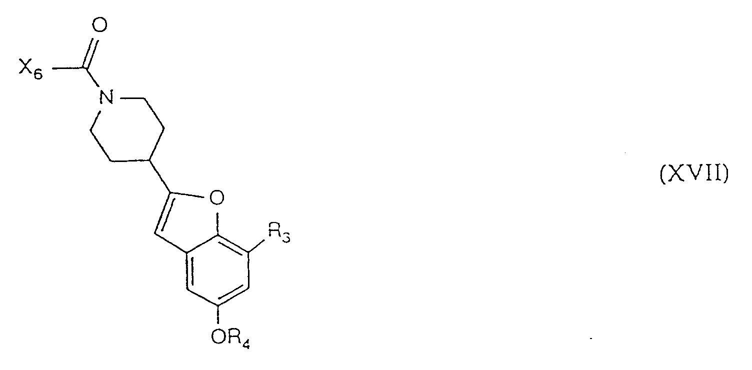
j) en ce que l'on clive sur celui-ci le groupe de formule -C(=O)-X₆ et, si désiré, en ce que l'on transforme le composé libre obtenu en un sel d'addition d'acides ou le sel d'addition d'acides obtenu en un composé libre ou en un autre sel d'addition d'acides.
